# EUROPEAN PATENT SPECIFICATION

(11) **EP 3 364 971 B1**
(45) Date of publication and mention of the grant of the patent: **17.06.2020**
(21) Application number: 16788389.1
(22) Date of filing: 21.10.2016
(51) Int. Cl.: A61K 31/497, A61K 31/4439, A61K 31/444, A61K 31/506, A61K 31/517, A61K 31/519, A61P 35/00, A61P 35/02

(54) **HETEROCYCLIC PDK1 INHIBITORS FOR USE TO TREAT CANCER**
HETEROCYCLISCHE PDK1-INHIBITOREN ZUR VERWENDUNG ZUR BEHANDLUNG VON KREBS
INHIBITEURS DE PDK1 HÉTÉROCYCLIQUES DESTINÉS À ÊTRE UTILISÉS POUR TRAITER LE CANCER

(30) Priority: 23.10.2015 US 201562245606 P
(43) Date of publication of application: 29.08.2018
(73) Proprietor: Sunesis Pharmaceuticals, Inc., South San Francisco, CA 94080 (US)
(72) Inventor: HANSEN, Stig K., Kensington, California 94708 (US); BINNERTS, Minke E., San Francisco, California 94110 (US)
(74) Representative: Cooley (UK) LLP
(86) International application number: PCT/US2016/058255
(87) International publication number: WO 2017/070565

(56) References cited:
- WO-A1-2011/044157

## Description

### CROSS REFERENCE TO RELATED APPLICATIONS

This application claims priority to U.S. provisional patent application no. 62/245,606, filed October 23, 2015.

### BACKGROUND

The 3-phosphoinositide-dependent protein kinase-1 (PDK1, also known as PDPK1) is a master kinase that activates other kinases important in cell growth and survival including members of the Akt (protein kinase B), PKC, RSK (S6K), and SGK families. PDK1 activates substrate kinases via activation T-loop phosphorylation (Belham et al., Curr. Biol., 1999, 9:R93-R96).

PDK1 is a 556-amino acid protein that consists of an N-terminal kinase (catalytic) domain, and a C-terminal pleckstrin homology (PH) domain. The PH domain interacts with phosphatidylinositol (PI) (3,4)-bisphosphate and phosphatidylinositol (3,4,5)-trisphosphate, contributing to localization and activation of certain PDK1 substrates, notably including Akt. The activation of Akt is believed to require a proper orientation of the kinase and PH domains of PDK1 and Akt at the membrane. Akt is itself known to be associated with cancers (Manning et al., Cell, 2007, 129(7):1261-1274), and is frequently mutated or hyperactivated in human cancers.

However, while PDK1 can interact with certain of its substrates through this PI-dependent (PH-mediated) mechanism, it can interact with other substrates through a distinct PI-independent mechanism. The N-terminal kinase domain has three ligand binding sites; a substrate binding site, an ATP binding site, and a docking site (also known as PIF pocket) for interaction with substrates. This docking site is known as the "PIF pocket," referring to its binding to a region of protein kinase C-related kinase-2 (PRK2), termed the PDK1-interacting fragment (PIF) (Biondi et al., EMBO J., 2000, 19(5):979-988). Several PDK1 substrates including S6K and Protein kinase C, require binding at this PIF pocket docking site.

As noted, PDK1 is important in regulating the activity of other kinases. Principal targets of PDK1 are the AGC subfamily of protein kinases (Alessi et al., Biochem. Soc. Trans, 2001, 29(2):1-14), such as isoforms of protein kinase B (PKB, also known as Akt), p70 ribosomal S6 kinase (S6K) (Avruch et al., Prog. Mol. Subcell. Biol., 2001, 26:115), p90 ribosomal S6 kinases (RSK1-4) (Frodin et al., EMBO J., 2000, 19:2924-2934), IKK and members of the protein kinase C (PKC) family (Le Good et al., Science, 1998, 281:2042-2045). PDK1-mediated signaling increases in response to insulin, growth factors, and extracellular matrix cell binding (integrin signaling) resulting in diverse cellular events such as cell survival, growth, proliferation, and glucose regulation (Lawlor et al., J. Cell Sci., 2001, 114:2903-2910; Lawlor et al., EMBO J., 2002, 21:3728-3738). Of the several PDK1 substrates mentioned above, much attention has focused on AKT. Development of potent and selective AKT inhibitors has been challenging and only two compounds have made it into clinical development: AZD5363 and MK2206. These compounds have shown promising anti-cancer activity in certain tumor types. However, more recent studies using these compounds have revealed, surprisingly, that many tumor types are not sensitive to AKT inhibition or express no or little activated AKT.

PDK1 is the only kinase known to phosphorylate Thr306 in the activation loop of AKT that is critical for activation of AKT kinase. Thus, PDK1 plays a critical role in AKT activation. Efforts to develop potent and selective PDK1 inhibitors with suitable drug like properties have been unsuccessful and no compounds have entered clinical development. Reported pre-clinical studies with PDK1 inhibitors GSK2334470 and BX-320/-795 have shown moderate efficacy and thus, it has been proposed that PDK1 may not be rate limiting in promoting cancer cell growth. Alternatively, these inhibitors may have poor pharmacological properties, failing to achieve sufficient inhibition to produce an effect, or the cancers cells used did not depend on PDK1 for growth.

The tumor-suppressor phosphatase with tensin homology (PTEN) is an important negative regulator of the cell-survival signaling pathway initiated by phosphatidylinositol 3-kinase (PI3K). The PDK1/Akt pathway is activated in many cancers via mutations in Receptor Tyrosine Kinases (RTKs), Ras, PI-3 kinase, or PTEN (Cully et al., Nature Reviews Cancer, 2006, 6:184-192). Elevated PDK1 activation and signaling has been detected in several cancers resulting from distinct genetic events such as PTEN mutations or over-expression of certain key regulatory proteins (Graff, Expert Opin. Ther. Targets, 2002, 6:103-113, Brognard et al., Cancer Res., 2001, 61:3986-3997). In fact, PTEN is one of the most frequently mutated genes in human cancer. PDK1 has been found to be overexpressed in acute myeloid leukemia (Zabkiewicz et al., Haematologica, 2014, 99(5):858-864). The potential of PDK1 inhibitors as anti-cancer compounds was indicated by transfection of a PTEN negative human cancer cell line (U87MG) with antisense oligonucleotides directed against PDK1. The resulting decrease in PDK1 protein levels led to a reduction in cellular proliferation and survival (Flynn et al., Curr. Biol., 2000, 10:1439-1442).

RSK2 (p90RSK2) is one of four ribosomal S6 kinases (S6K) known in humans, a family of serine/threonine kinases that are activated by the MAPK/ERK pathway. RSK comprises two kinase domains: the C-terminal domain autophosphorylates RSK2, which is necessary for its activation; the N-terminal domain of activated RSK2 phosphorylates downstream substrates such as certain transcriptional regulators. It is possible that RSK2 plays a key role in tumors that are not dependent on AKT or provides a key resistance mechanism to bypass AKT signaling upon treatment with AKT inhibitors.

RSK2 is known to be activated through phosphorylation by PDK1 through the PI-independent, PIF pocket mechanism, and promotes cellular proliferation in various cell types, and may contribute to certain cancers. For example, RSK2 has been shown to be activated in certain forms of myeloid leukemia. Inhibition of RSK2 induced apoptotic cell death in Molm14 and Mv(4;11) leukemia cells and primary samples from AML patients, but failed to affect apoptosis in Ba/F3 or K562 cells or in primary samples from CML patients (Elf et al., Blood, 2011, 117(25):6885-6894). Separately, it has been reported that RSK2 inhibition induced apoptosis in certain myeloma cells, and that receptor tyrosine kinase fibroblast growth factor receptor 3 (FGFR3) activates RSK2, which may induce hematopoietic transformation (Kang et al., J. Biol. Chem., 2008, 283(8):4652-4657; Kang et al., Mol. Cell. Biol., 2009, 29(8):2105-2117).

Consequently, there is a need for effective inhibitors of PDK1 with differential pharmacological and therapeutic characteristics. The present invention fulfills these and other needs.

### SUMMARY OF THE INVENTION

It has now been found that certain compounds impair or block PI-independent, PIF pocket mediated substrate binding and have broad anti-tumor activity in hematologic cancers and other cancers. On the one hand, it has now been found that these compounds appear to modify the conformation of PDK1 to block PIF binding, thereby preventing the binding and phosphorylation of PI-independent (PIF-dependent) substrates, while yet inhibiting PDK1 kinase activity by also blocking ATP binding. This dual-mechanism function may by critical to effectively inhibit PDK1 signaling by affecting both PI-dependent and PI-independent substrate phosphorylation. This function, therefore, could make these compounds useful in treatment of cancers that are Akt-independent or in which resistance to Akt inhibitors arises. In addition, such dual-mechanism inhibitors may have utility in treatment of cancers that are dependent for growth on RSK2 activity or other PIF-dependent substrates downstream of PDK1, whether or not AKT is active.

Described herein are methods which employ compounds of Formula **I:** 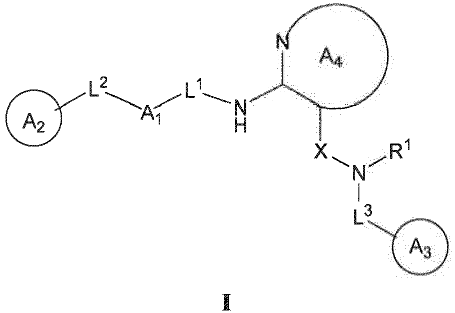 or a pharmaceutically acceptable salt thereof, in which each of A₁, Ring A₂, Ring A₃, Ring A₄, L¹, L², L³, X, and R¹ are as defined and described in classes and subclasses herein. Such compounds are useful as modulators of cellular survival pathways implicating certain protein kinases (e.g., PDK1, RSK2, Akt), and thus are useful, for example, for the treatment of PDK1-, RSK2-, and Akt-mediated diseases.

Also described herein are pharmaceutical compositions comprising a compound of Formula I as described, in which the compound is present in an amount effective to inhibit a PDK1-PIF mediated substrate interaction-dependent cancer survival pathway, such as an RSK2-dependent pathway, or an Akt-independent pathway, that is implicated in cancer growth and survival. Also described herein are pharmaceutical compositions comprising a compound of Formula I and optionally further comprising an additional therapeutic agent. The additional therapeutic agent may be an agent for the treatment of cancer.

The present invention provides a compound selected from the group consisting of: 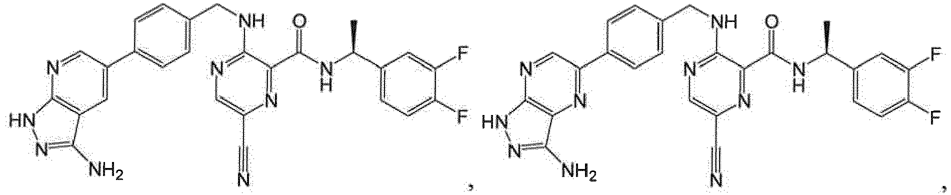 and 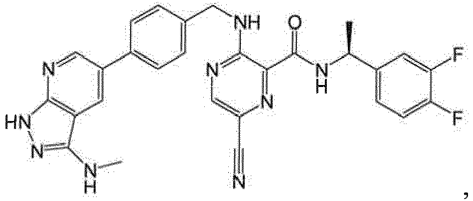 and pharmaceutically acceptable salts thereof, for use in a method of treating cancer in a subject in need thereof, in which cancer growth or survival is dependent on a PDK1-PIF-mediated substrate interaction, the method comprising administering to said subject a therapeutically effective amount of the compound or pharmaceutically acceptable salt thereof. In some embodiments, the cancer is a hematologic cancer selected from the group consisting of leukemias, lymphomas, and myelomas. In some embodiments, the hematologic cancer is selected from anaplastic large-cell lymphoma, non-Hodgkin's lymphoma, Hodgkin's lymphoma, B-cell lymphoma, T-cell lymphoma, mantle cell lymphoma, histiocytic lymphoma, T-cell leukemia, chronic lymphocytic leukemia, multiple myeloma, chronic myelogenous leukemia, acute lymphocytic (lymphoblastic) leukemia, acute myelogenous leukemia, acute myeloblastic leukemia, and plasma cell leukemia.

The present invention also provides a pharmaceutical composition for use in treating cancer in a subject, in which the growth or proliferation of the cancer is dependent on a PDK1-PIF-mediated substrate interaction, comprising a formulation including a compound selected from the group consisting of: 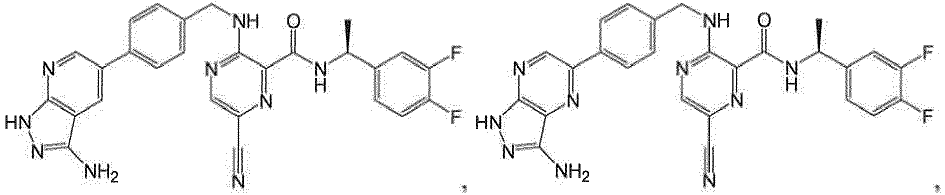 and 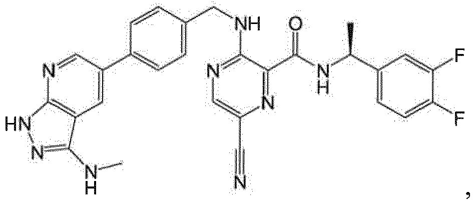 and pharmaceutically acceptable salts thereof; and a pharmaceutically acceptable carrier.

The present invention also provides a pharmaceutical composition for use in a combinational therapy of treating cancer in a subject, comprising a formulation including a compound selected from the group consisting of: 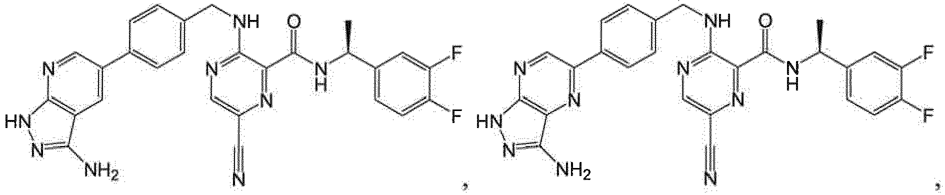 and 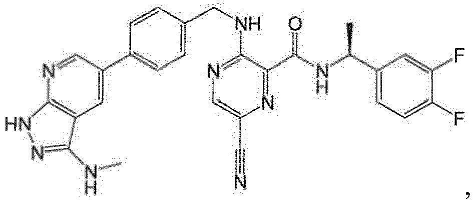 and pharmaceutically acceptable salts thereof; and a pharmaceutically acceptable carrier, wherein the combinational therapy further comprises an effective amount of a second anti-cancer agent.

Also described herein are methods for inhibiting a kinase activation pathway implicated in cancer growth and survival in a patient or a biological sample, comprising administering to said patient, or contacting said biological sample with, an effective inhibitory amount of a compound of Formula I. Also described herein are methods for treating any disorder involving such a kinase activation pathway, comprising administering to a subject in need thereof a therapeutically effective amount of a compound of Formula I. Such methods are described in detail herein.

### BRIEF DESCRIPTION OF THE DRAWINGS

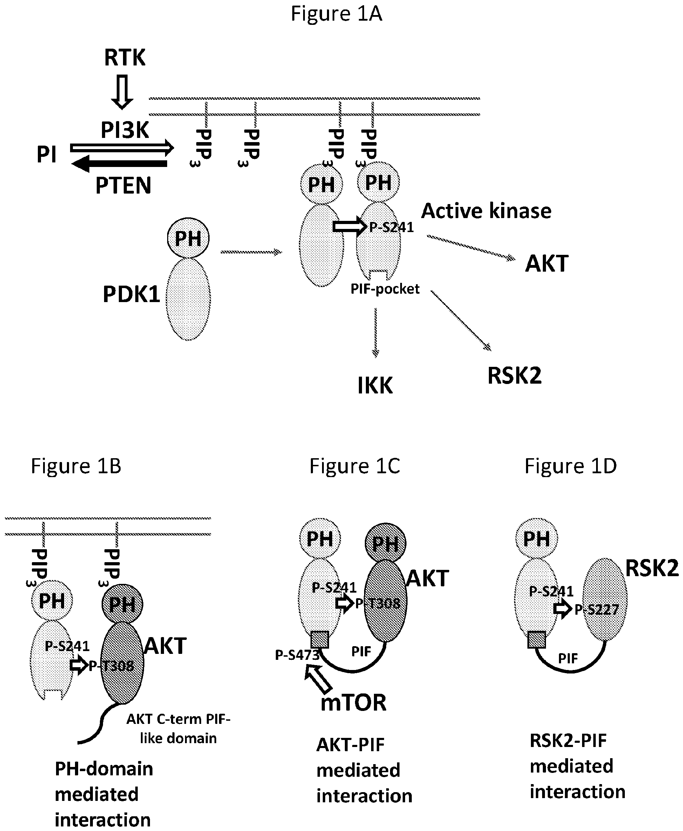

Figure 1A shows the interactions among elements of phosphatidyl-inositol (PI)-dependent (PH-mediated) or PI-independent (PIF-mediated) cellular pathways; Figure 1B shows PH-domain interactions between PDK1 and Akt; Figure 1C shows PIF-mediated interaction between PDK1 and Akt; Figure ID shows PIF-mediated interaction between PDK1 and RSK2.

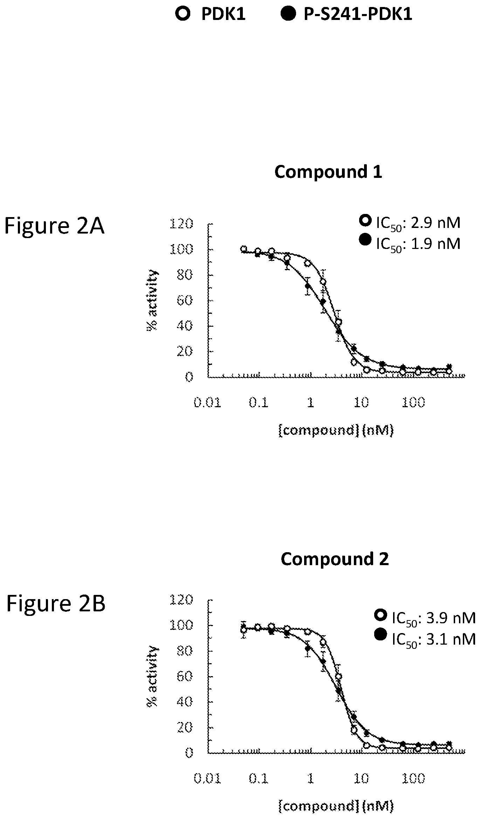

Figures 2A and 2B shows PDK1 kinase activity inhibition curves for representative compounds of Formula I: Compound 1 (Figure 2A) and Compound 2 (Figure 2B).

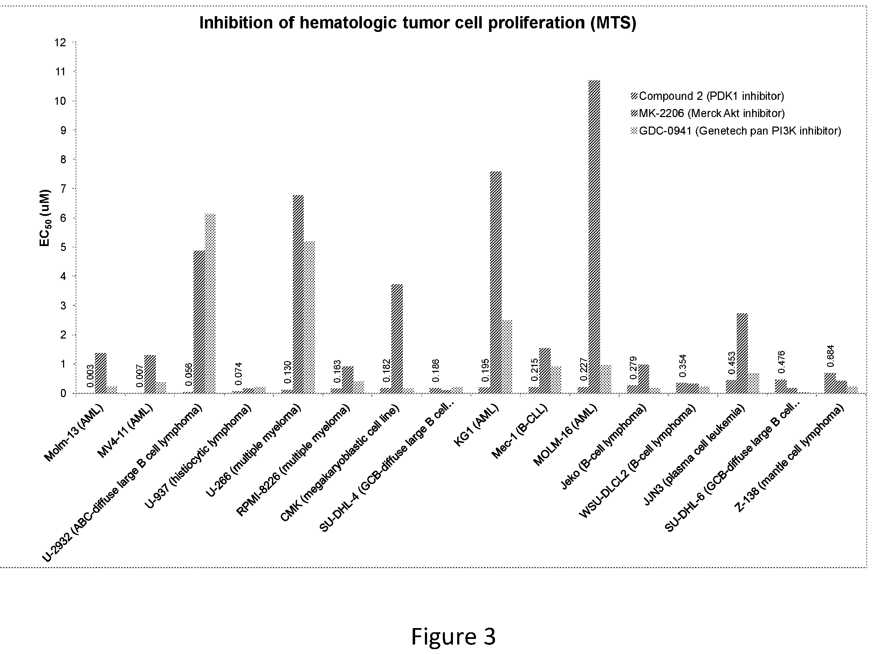

Figure 3 shows inhibition of proliferation of hematologic tumor cell lines in vitro by test compounds.

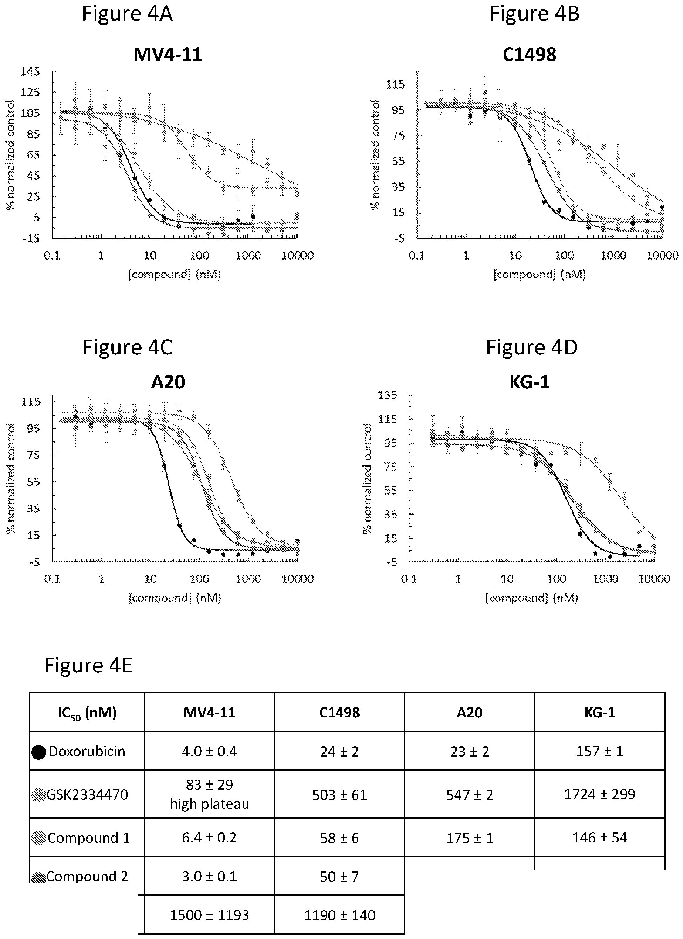

Figures 4A-4D shows growth inhibition in several hematologic tumor cell lines by test compounds: MV4-11 (Figure 4A), C1498 (Figure 4B), and A20 (Figure 4C). Figure 4D provides a key for Figures 4A-4C, and provides IC₅₀ data for the compounds in each cell line.

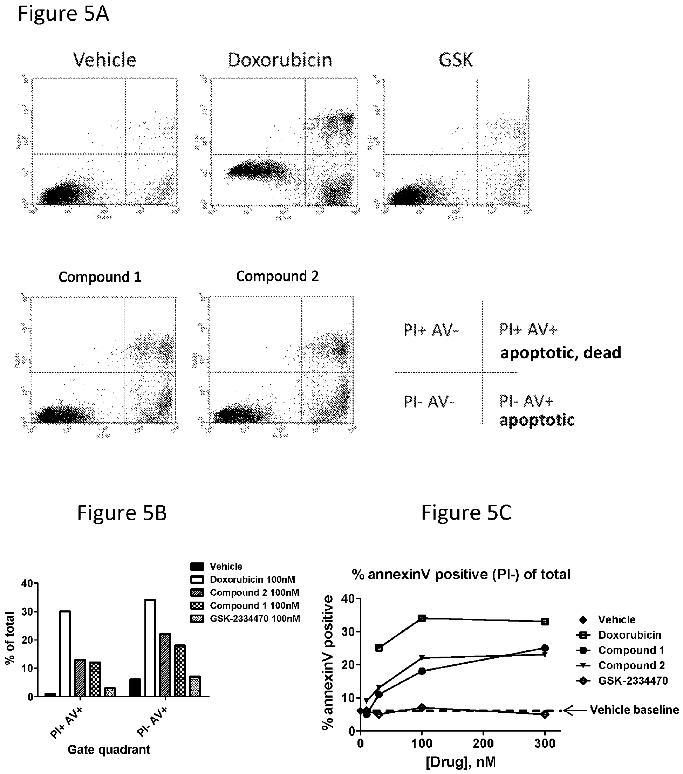

Figure 5A shows FACS dotplots of MV4-11 cells treated with vehicle or a test compound. Parameters are annexin V (AV; horizontal axis) against propidium iodide (PI; vertical axis); Figure 5B shows the percent of total cells in the gate quadrants for PI+AV+ and PI-AV+ of the plots in Figure 5A; Figure 5C shows the dose-response relationships of the test compounds' capacity to induce apoptosis as measured by the cells in the PI-AV+ quadrants of the plots in Figure 5A.

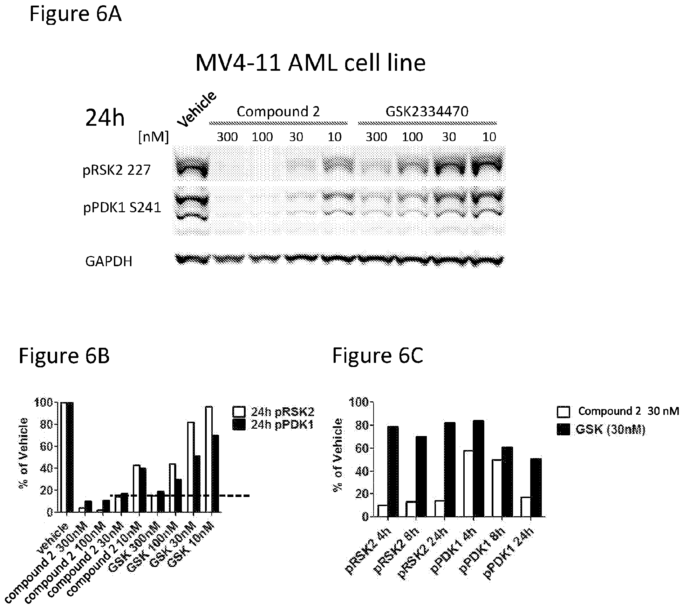

Figure 6A shows a Western blot of phosphorylated RSK2 (pRSK2) and phosphorylated PDK1 (pPDK1) levels at various concentrations of test compounds; Figure 6B shows the amounts (quantification of 6A normalized to GAPDH) of pRSK2 and pPDK1 detected at 24 hours exposure to various concentrations of test compounds, expressed as a percentage of the respective phosphorylated proteins detected in control samples; Figure 6C shows the amounts of pRSK2 and pPDK1 detected based on exposure to 30 nM test compounds for various times, expressed as a percentage of the respective phosphorylated proteins detected in control samples.

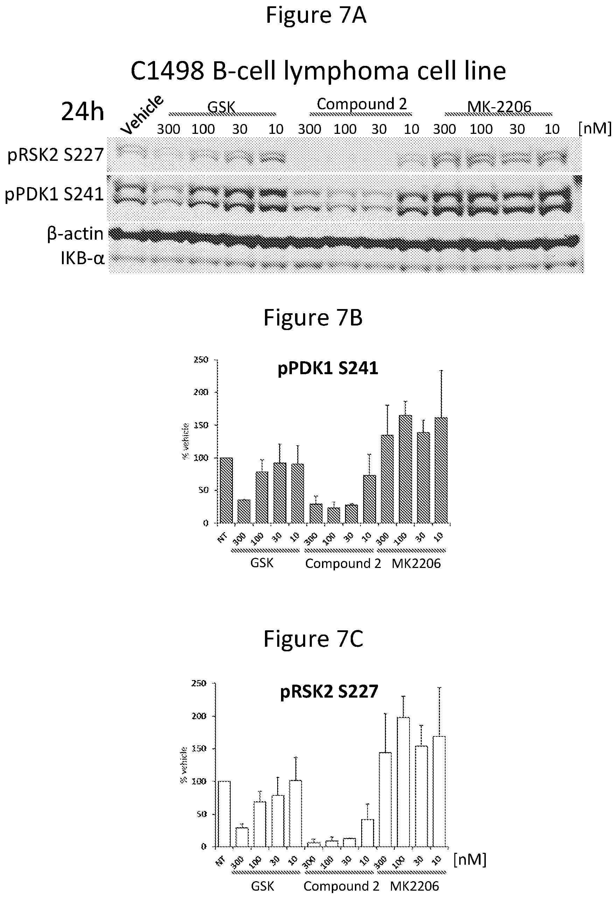

Figure 7A shows a Western blot of phosphorylated RSK2 (pRSK2) and phosphorylated PDK1 (pPDK1) levels at various concentrations of three test compounds; Figures 7B and 7C show the amounts (quantification of 7A normalized to GAPDH) of pPDK1 and pRSK2 detected after exposure to various concentrations of test compounds, expressed as a percentage of the respective phosphorylated proteins detected in control samples.

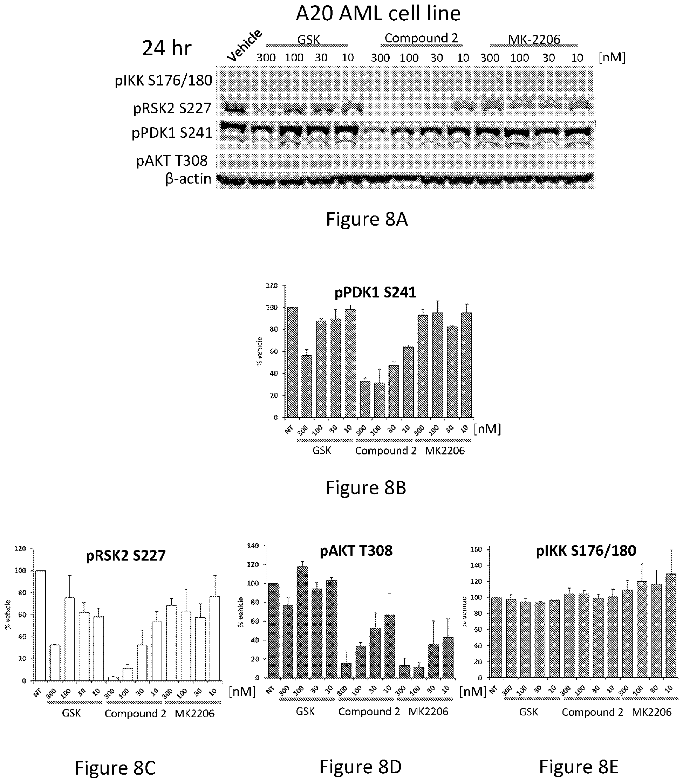

Figure 8A shows a Western blot of phosphorylated RSK2 (pRSK2), phosphorylated PDK1 (pPDK1), phosphorylated Akt (pAkt), and phosphorylated IKK (pIKK) levels at various concentrations of three test compounds; Figures 8B through 8E show the amounts (quantification of 8A normalized to GAPDH) of pPDK1, pRSK2, pAkt, and pIKK detected after exposure to various concentrations of test compounds, expressed as a percentage of the respective phosphorylated proteins detected in control samples.

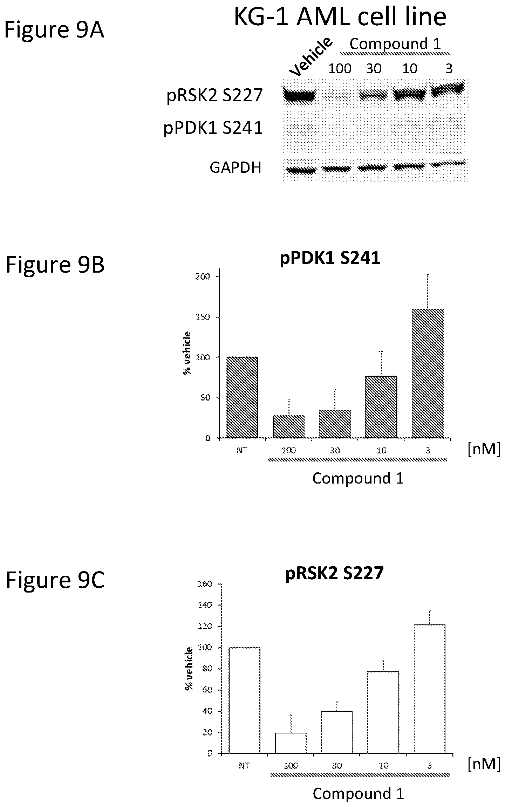

Figures 9A-9C depict KG-1 cell line sensitivity to Compound 1, and inhibition of pRSK2 and pPDK1 levels at 100 nM compound concentration.

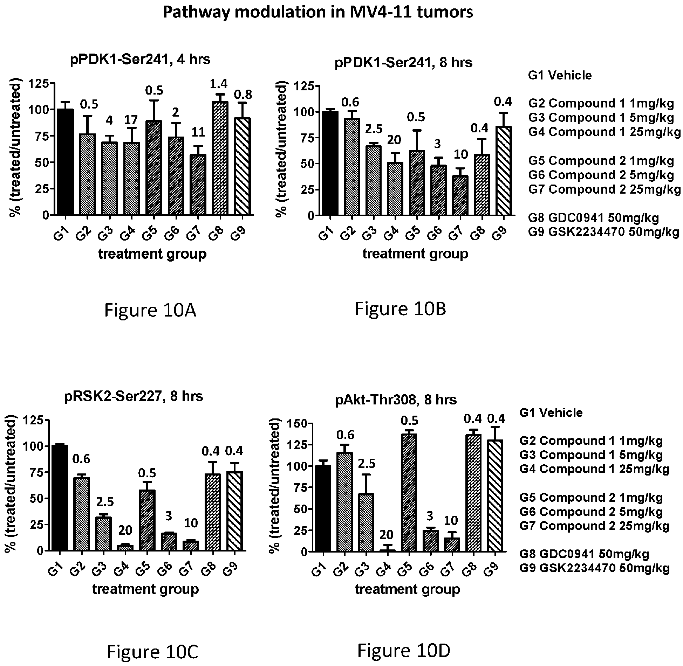

Figures 10A-10D show exemplary data obtained from quantification of Western blot analyses of tumor samples from MV4-11 tumor xenografts after a single dose of compound. Figures 10A and 10B show pPDK1 levels following 4- and 8-hour exposure to test compounds, expressed as a percentage of levels detected in control samples. Figures 10C and 10D show pRSK2 levels and pAkt levels following 8-hour exposure to test compounds, expressed as a percentage of levels detected in control samples. The numbers above the columns indicate the concentration of compound (mM) present in tumors as determined by LC-MS/MS.

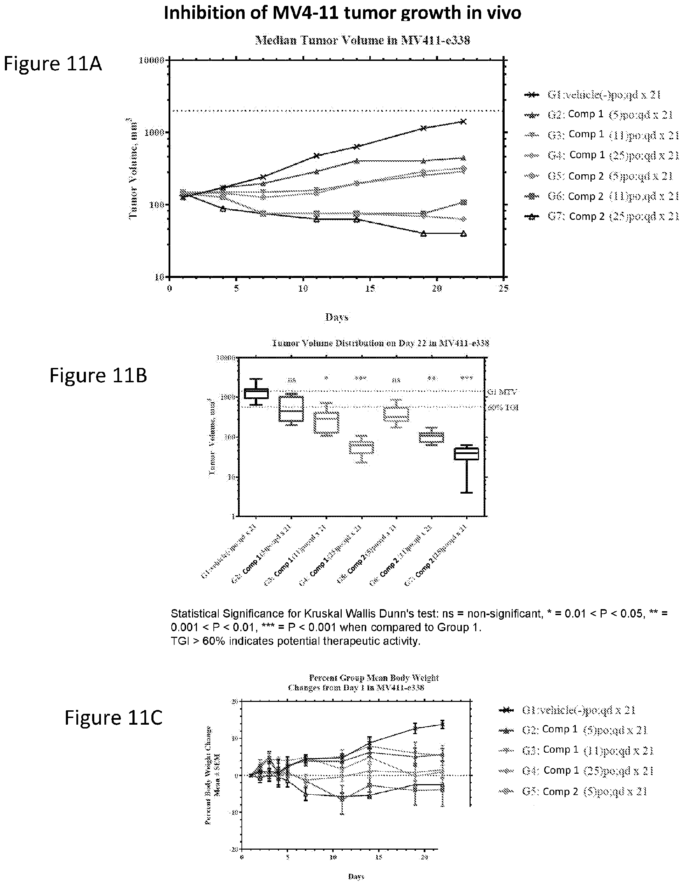

Figure 11A shows median MV4-11 tumor volume as a function of time for various treatment groups exposed to compounds of Formula I in a murine xenograft model; Figure 11B shows tumor volue distribution across treatment groups; Figure 11C shows percent group mean body weight as a function of time for various treatment groups.

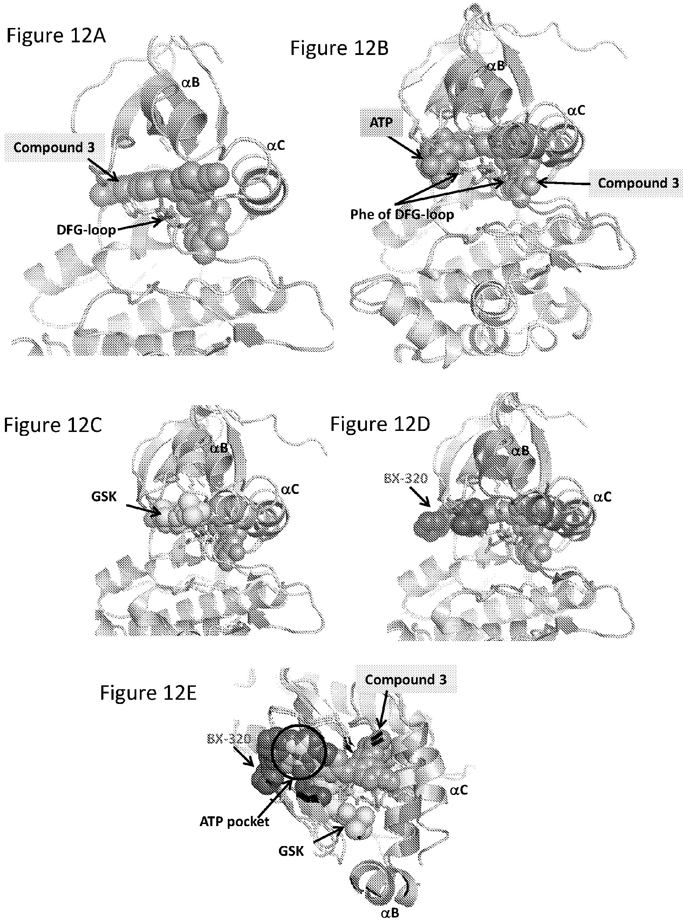

Figure 12A shows a three-dimensional representation of a cocrystal in which Compound 3 is bound to PDK1; Figure 12B shows a comparative three-dimensional representation of cocrystals in which Compound 3 (lighter grey) or ATP (darker grey) is bound to PDK1; Figure 12C shows a comparative three-dimensional representation of a cocrystal in which Compound 3 (darker grey) or GSK2334470 (lighter grey) is bound to PDK1; Figure 12D shows a comparative three-dimensional representation of a cocrystal in which Compound 3 (lighter grey) or BX-320 (darker grey) is bound to PDK1; Figure 12E shows a comparative three-dimensional representation of cocrystals in which Compound 3 (medium grey), GSK2334470 (lightest grey), or BX-320 (darkest grey) is bound to PDK1.

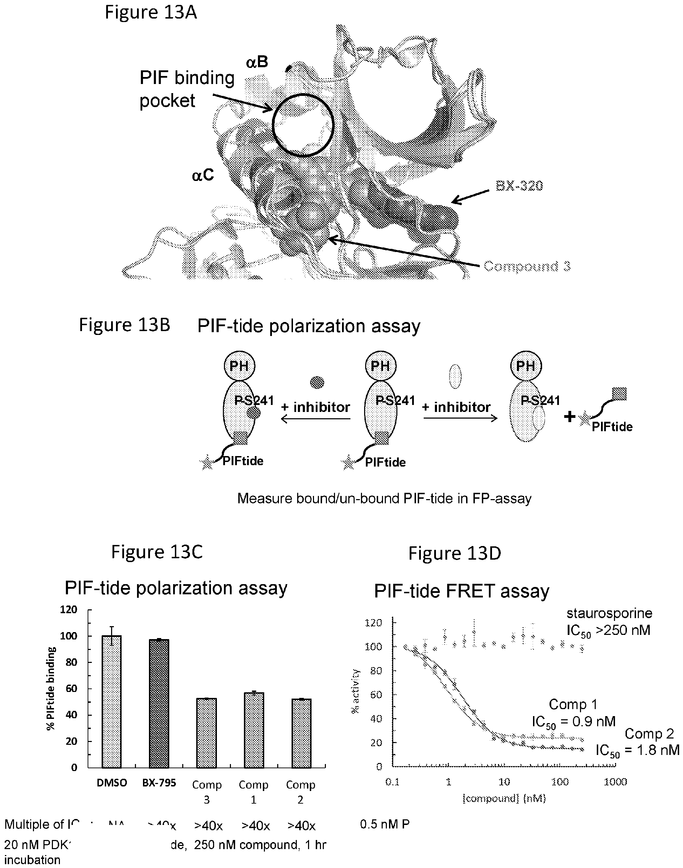

Figure 13A shows three-dimensional representation of cocrystals in which Compound 3 (medium grey), GSK2334470 (lightest grey), or BX-320 (darkest grey) is bound to PDK1; Figure 13B illustrates the conceptual outline of a PIF-tide binding assay in which comparative activity of PIF-tide blocking of test compounds may be assessed; Figure 13C shows measured PIF-tide binding by PDK1 in the presence and absence of test compounds, expressed as a percentage of DMSO control.

### DETAILED DESCRIPTION OF CERTAIN EMBODIMENTS

### Compounds Useful in Methods of the Invention

PDK1 can interact with its substrates through phosphatidyl-inositol (PI)-dependent (PH-mediated) or PI-independent (PIF-mediated) mechanisms. Here we describe a family of compounds that occupy both the ATP-binding pocket and the adaptive ("allosteric") pocket and block PI-independent substrate binding and have anti-tumor activity in solid tumors and hematologic cancers. Compounds of Formula I, as described below, have a distinct activity profile, which manifests in the ability to impair the growth and survival of cancer cells, such as cells that are resistant to Akt inhibition, or that are dependent on RSK2 activity.

Thus, described herein are methods of use of a compound of Formula **I**: 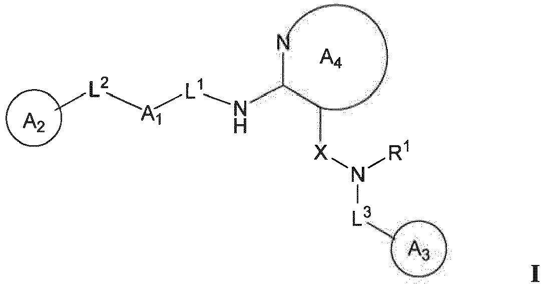 or a pharmaceutically acceptable salt thereof, in which:
R¹ is hydrogen or optionally substituted C₁₋₆ aliphatic, or:
   R¹ and a substituent on Ring A₄ are taken together with their intervening atoms to form an optionally substituted 5-7 membered partially unsaturated or aromatic fused ring having 1-3 heteroatoms independently selected from nitrogen, oxygen, or sulfur;
X is -C(O)- or -S(O)₂-,
L¹ is a covalent bond or an optionally substituted bivalent group selected from C₁₋₄ alkylene, C₂₋₄ alkenylene, or C₂₋₄ alkynylene wherein one or more methylene units of L¹ are optionally and independently replaced by -Cy¹-, -O-, -S-, -N(R²)-, -C(O)-, -C(O)N(R²)-, -N(R²)C(O)N(R²)-, -N(R²)C(O)-, -N(R²)C(O)O-, -OC(O)N(R²)-, -S(O)₂-, -S(O)₂N(R²)-, -N(R²)S(O)₂-, -OC(O)-, or -C(O)O-;
Cy¹ is an optionally substituted bivalent ring selected from phenylene, 3-7 membered saturated or partially unsaturated carbocyclylene, 4-7 membered saturated or partially unsaturated heterocyclylene having 1-2 heteroatoms independently selected from nitrogen, oxygen, or sulfur, or 5-6 membered heteroarylene having 1-3 heteroatoms independently selected from nitrogen, oxygen, or sulfur;
each R² is hydrogen or optionally substituted C₁₋₆ aliphatic;
A₁ is a covalent bond or an optionally substituted bivalent ring selected from 3-7 membered saturated or partially unsaturated monocyclic carbocyclylene, 7-10 membered saturated or partially unsaturated bicyclic carbocyclylene, 4-7 membered saturated or partially unsaturated monocyclic heterocyclylene having 1-2 heteroatoms independently selected from nitrogen, oxygen, or sulfur, 7-10 membered saturated or partially unsaturated bicyclic heterocyclylene having 1-3 heteroatoms independently selected from nitrogen, oxygen, or sulfur, phenylene, 8-10 membered bicyclic arylene, 5-6 membered monocyclic heteroarylene having 1-3 heteroatoms independently selected from nitrogen, oxygen, or sulfur, or 8-10 membered bicyclic heteroarylene having 1-4 heteroatoms independently selected from nitrogen, oxygen, or sulfur;
L² is a covalent bond, alkylidenylene, or an optionally substituted alkylene chain in which one or more methylene units of L² are optionally and independently replaced by -O-, -S-, -N(R²)-, -C(O)-, -C(O)N(R²)-, -N(R²)C(O)N(R²)-, -N(R²)C(O)-, -N(R²)C(O)O-, -OC(O)N(R²)-, -S(O)₂-, -S(O)₂N(R²)-, -N(R²)S(O)₂-, -OC(O)-, or -C(O)O-;
Ring A₂ is a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered monocyclic heteroaryl ring having 1-3 heteroatoms independently selected from nitrogen, oxygen, or sulfur, an 8-10 membered bicyclic heteroaryl ring having 1-4 heteroatoms independently selected from nitrogen, oxygen, or sulfur, or a 10-16 membered saturated, partially unsaturated, or aromatic tricyclic ring having 0-4 heteroatoms independently selected from nitrogen, oxygen, or sulfur, wherein Ring A₂ is optionally substituted with 1-4 R^{x} groups;
each R^{x} is independently -R, optionally substituted alkylidenyl, oxo, halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂;
each R is independently hydrogen or an optionally substituted group selected from CI-6 aliphatic, a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur;
each R' is independently -R, or two R' groups on the same nitrogen are taken together with their intervening atoms to form an optionally substituted 5-8 membered saturated, partially unsaturated, or aromatic ring having 1-4 heteroatoms independently selected from nitrogen, oxygen, or sulfur;
L³ is a covalent bond or an optionally substituted C₁₋₄ alkylene chain in which one or more methylene units of L³ are optionally and independently replaced by -O-, -S-, -N(R²)-, -C(O)-, -C(O)N(R²)-, -N(R²)C(O)N(R²)-, -N(R²)C(O)-, -N(R²)C(O)O-, -OC(O)N(R²)-, -S(O)₂-, -S(O)₂N(R²)-, -N(R²)S(O)₂-, -OC(O)-, or -C(O)O-;
Ring A₃ is an optionally substituted ring selected from a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 810 membered bicyclic aryl ring, a 5-6 membered monocyclic heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur;
Ring A₄ is a 5-6 membered heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur; wherein any substitutable carbon on Ring A₄ is optionally substituted with R³, R⁴, or R⁵, and any substitutable nitrogen on Ring A₄ is optionally substituted with R⁶;
each of R³, R⁴, and R⁵ is independently -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂; or:
   R³ and R⁴ or R⁴ and R⁵ are taken together with their intervening atoms to form an optionally substituted fused ring selected from a 4-7 membered partially unsaturated carbocyclic ring, phenyl, a 5-6 membered partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur;
   each R⁶ is independently -R, -C(O)R, -CO₂R, -C(O)C(O)R, -C(O)CH₂C(O)R, -S(O)R, -S(O)₂R, -C(O)N(R')₂, or -S(O)₂N(R')₂; or:
      R³ and R⁶ are taken together with their intervening atoms to form an optionally substituted fused ring selected from a 5-6 membered saturated or partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur;
      provided that:
         when A₁ is a bivalent monocyclic ring and L¹ is a covalent bond, L² is not -O-;
         when A₁ is a bivalent monocyclic or bicyclic ring, L¹ and L² are not simultaneously a covalent bond; and
         L¹, A₁, and L² are not simultaneously a covalent bond.

For example, compounds of Formula I as described herein may be used to inhibit the growth, proliferation, or survival of cancer cells in which PDK1-PIF-mediated substrate interaction-dependent cell survival pathways are implicated.

Described herein is a method of treating cancer in a subject in need thereof by inducing cancer cell apoptosis through inhibition of PDK1-PIF mediated substrate interaction-dependent cancer survival pathways, comprising administering to said subject a therapeutically effective amount of a compound of Formula I as described herein.

Also described herein is a method of treating cancer in a subject in need thereof by inhibiting PDK1-PIF mediated substrate interaction-dependent cancer cell growth or proliferation, comprising administering to said subject a therapeutically effective amount of a compound of Formula I as described herein.

Also described herein is a method for inhibiting the growth or proliferation of cancer cells by inhibiting Akt-independent cancer cell growth or proliferation pathways dependent on PDK1-PIF mediated substrate interaction, the method comprising contacting the cancer cells with an effective amount of a compound of Formula I as described herein.

Also described herein is a method for inducing apoptosis of cancer cells by inhibiting Akt-independent cancer cell survival pathways dependent on PDK1-PIF mediated substrate interaction, the method comprising contacting the cancer cells with an effective amount of a compound of Formula I as described herein.

Also described herein is a method of inhibiting the growth or proliferation of cancer cells the growth or proliferation of which is dependent on PIF-mediated substrate binding by PDK1, the method comprising contacting the cancer cells with a compound of Formula I as described herein in an amount sufficient to inhibit growth or proliferation of the cancer cells.

Also described herein is a method of inducing apoptosis of cancer cells the growth or proliferation of which is dependent on PIF-mediated substrate binding by PDK1, the method comprising contacting the cancer cells with an effective amount of a compound of Formula I as described herein.

Also described herein is a method of inhibiting PIF-mediated substrate binding by PDK1 in cancer cells, comprising contacting the cells with a compound of Formula I, whereby growth or proliferation of the cancer cells is inhibited.

Also described herein is a method of inducing apoptosis in cancer cells, comprising contacting cancer cells with a compound of Formula I as described herein that inhibits PIF-mediated substrate binding by PDK1.

Also described herein is a method of preparing a medicament for use in the treatment of cancer whose growth or survival is dependent on a PDK1-PIF-mediated substrate interaction, comprising a therapeutically effective amount of a compound of Formula I as described herein and a pharmaceutically acceptable excipient.

Also described herein is a product comprising a container and a medicament for use in the treatment of cancer whose growth or survival is dependent on a PDK1-PIF-mediated substrate interaction, in which the medicament comprises a compound of Formula I as described herein and a pharmaceutically acceptable excipient.

Compounds of Formula I as described herein may be used to inhibit the growth, proliferation, or survival of cancer cells in which RSK2-dependent cell survival pathways are implicated.

Also described herein is a method of treating cancer in a subject in need thereof by inducing cancer cell apoptosis through inhibition of RSK2-dependent survival pathways, comprising administering to said subject a therapeutically effective amount of a compound of Formula I as described herein.

Also described herein is a method of treating cancer in a subject in need thereof by inhibiting RSK2-dependent cancer cell growth or proliferation, comprising administering to said subject a therapeutically effective amount of a compound of Formula I as described herein.

Also described herein is a method of inhibiting the growth or proliferation of cancer cells the growth or proliferation of which is dependent on kinase activity of RSK2, the method comprising contacting the cancer cells with a compound of Formula I as described herein in an amount sufficient to inhibit RSK2 activity in the cancer cells.

Also described herein is a method of inducing apoptosis in cancer cells, comprising contacting cancer cells with a compound of Formula I as described herein that inhibits RSK2 activation by PDK1.

Compounds of Formula I as described herein may be used to inhibit the growth, proliferation, or survival of cancer cells in which Akt-independent cell survival pathways are implicated. Such cells are considered to be resistant to inhibition of Akt activity or inhibition of the activity of Akt-mediated survival pathways. Thus cells that can survive even if Akt is substantially inactive, or that are resistant to, or do not respond to, Akt inhibitors, may yet be inhibited by compounds of Formula I as described herein.

Also described herein is a method of treating cancer in a subject in need thereof by inducing cancer cell apoptosis through inhibition of Akt-independent cancer cell survival pathways, comprising administering to said subject a therapeutically effective amount of a compound of Formula I as described herein.

Also described herein is a method of treating cancer in a subject in need thereof by inhibiting Akt-independent cancer cell growth or proliferation, comprising administering to said subject a therapeutically effective amount of a compound of Formula I as described herein.

Also described herein is a method of inhibiting the growth or proliferation of cancer cells the growth or proliferation of which is not dependent on kinase activity of Akt, the method comprising contacting the cancer cells with a compound of Formula I as described herein in an amount sufficient to inhibit growth or proliferation of the cancer cells.

Also described herein is a method of inducing apoptosis of cancer cells the growth or proliferation of which is not dependent on kinase activity of Akt, the method comprising contacting the cancer cells with an effective amount of a compound of Formula I as described herein.

Also described herein is a method of inducing apoptosis in cancer cells in which viability is Akt-independent, comprising contacting the cancer cells with an amount of a compound of Formula I as described herein that is effective to interfere with PIF-mediated substrate binding by PDK1 in the cancer cells.

Also described herein is a method of inhibiting Akt-independent growth or proliferation of cancer cells, comprising contacting the cancer cells with an effective amount of a compound of Formula I as described herein.

Also described herein is a method treating a subject having a cancer the growth or proliferation of which is Akt-independent, comprising administering to the subject an amount of a compound of Formula I as described herein that is effective to impair growth or proliferation of the cancer.

Also described herein is a method of inducing apoptosis in cancer cells in which viability is RSK2-dependent or Akt-independent, comprising contacting the cancer cells with an amount of a compound of Formula I as described herein that is effective to interfere with PIF-mediated substrate binding by PDK1 in the cancer cells.

Also described herein is a method of inducing apoptosis of cancer cells the growth or proliferation of which is dependent on PDK1 PIF-binding activity, the method comprising contacting the cancer cells with an effective amount of a compound of Formula I as described herein.

Also described herein is a method of inducing apoptosis of cancer cells the growth or proliferation of which is dependent on PDK1 PIF-binding activity, the method comprising contacting the cancer cells with an effective amount of a compound of Formula I as described herein.

Also described herein is a method of inducing apoptosis of cancer cells the growth or proliferation of which is dependent on RSK2 activity, the method comprising contacting the cancer cells with an effective amount of a compound of Formula I as described herein.

Also described herein are methods which use a compound of Formula **Ia:** 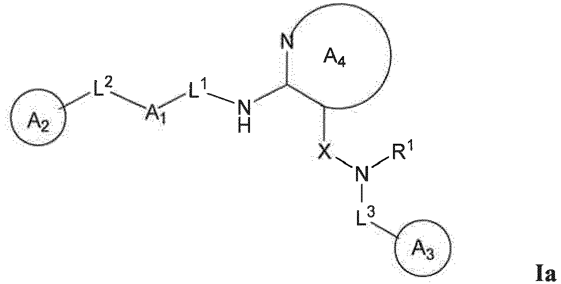 or a pharmaceutically acceptable salt thereof, in which:
R¹ is hydrogen or optionally substituted C₁₋₆ aliphatic, or:
   R¹ and a substituent on Ring A₄ are taken together with their intervening atoms to form an optionally substituted 5-7 membered partially unsaturated or aromatic fused ring having 1-3 heteroatoms independently selected from nitrogen, oxygen, or sulfur;
X is -C(O)- or -S(O)₂-,
L¹ is a covalent bond or an optionally substituted bivalent group selected from C₁₋₄ alkylene, C₂₋₄ alkenylene, or C₂₋₄ alkynylene in which one or more methylene units of L¹ are optionally and independently replaced by -Cy¹-, -O-, -S-, -N(R²)-, -C(O)-, -C(O)N(R²)-, -N(R²)C(O)N(R²)-, -N(R²)C(O)-, -N(R²)C(O)O-, -OC(O)N(R²)-, -S(O)₂-, -S(O)₂N(R²)-, -N(R²)S(O)₂-, -OC(O)-, or -C(O)O-;
Cy¹ is an optionally substituted bivalent ring selected from phenylene, 3-7 membered saturated or partially unsaturated carbocyclylene, 4-7 membered saturated or partially unsaturated heterocyclylene having 1-2 heteroatoms independently selected from nitrogen, oxygen, or sulfur, or 5-6 membered heteroarylene having 1-3 heteroatoms independently selected from nitrogen, oxygen, or sulfur;
each R² is hydrogen or optionally substituted C₁₋₆ aliphatic;
A₁ is a covalent bond or an optionally substituted bivalent ring selected from 3-7 membered saturated or partially unsaturated monocyclic carbocyclylene, 7-10 membered saturated or partially unsaturated bicyclic carbocyclylene, 4-7 membered saturated or partially unsaturated monocyclic heterocyclylene having 1-2 heteroatoms independently selected from nitrogen, oxygen, or sulfur, 7-10 membered saturated or partially unsaturated bicyclic heterocyclylene having 1-3 heteroatoms independently selected from nitrogen, oxygen, or sulfur, phenylene, 8-10 membered bicyclic arylene, 5-6 membered monocyclic heteroarylene having 1-3 heteroatoms independently selected from nitrogen, oxygen, or sulfur, or 8-10 membered bicyclic heteroarylene having 1-4 heteroatoms independently selected from nitrogen, oxygen, or sulfur;
L² is a covalent bond, alkylidenylene, or an optionally substituted alkylene chain in which one or more methylene units of L² are optionally and independently replaced by -O-, -S-, -N(R²)-, -C(O)-, -C(O)N(R²)-, -N(R²)C(O)N(R²)-, -N(R²)C(O)-, -N(R²)C(O)O-, -OC(O)N(R²)-, -S(O)₂-, -S(O)₂N(R²)-, -N(R²)S(O)₂-, -OC(O)-, or -C(O)O-;
Ring A₂ is a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered monocyclic heteroaryl ring having 1-3 heteroatoms independently selected from nitrogen, oxygen, or sulfur, an 8-10 membered bicyclic heteroaryl ring having 1-4 heteroatoms independently selected from nitrogen, oxygen, or sulfur, or a 10-16 membered saturated, partially unsaturated, or aromatic tricyclic ring having 0-4 heteroatoms independently selected from nitrogen, oxygen, or sulfur, in which Ring A₂ is optionally substituted with 1-4 R^{x} groups;
each R^{x} is independently -R, optionally substituted alkylidenyl, oxo, halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂;
each R is independently hydrogen or an optionally substituted group selected from C₁₋₆ aliphatic, a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur;
each R' is independently -R, or two R' groups on the same nitrogen are taken together with their intervening atoms to form an optionally substituted 5-8 membered saturated, partially unsaturated, or aromatic ring having 1-4 heteroatoms independently selected from nitrogen, oxygen, or sulfur;
L³ is a covalent bond or an optionally substituted C₁₋₄ alkylene chain in which one or more methylene units of L³ are optionally and independently replaced by -O-, -S-, -N(R²)-, -C(O)-, -C(O)N(R²)-, -N(R²)C(O)N(R²)-, -N(R²)C(O)-, -N(R²)C(O)O-, -OC(O)N(R²)-, -S(O)₂-, -S(O)₂N(R²)-, -N(R²)S(O)₂-, -OC(O)-, or -C(O)O-;
Ring A₃ is an optionally substituted ring selected from a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered monocyclic heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur;
Ring A₄ is a 5-6 membered heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur; in which any substitutable carbon on Ring A₄ is optionally substituted with R³, R⁴, or R⁵, and any substitutable nitrogen on Ring A₄ is optionally substituted with R⁶;
each of R³, R⁴, and R⁵ is independently -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂; or:
   R³ and R⁴ or R⁴ and R⁵ are taken together with their intervening atoms to form an optionally substituted fused ring selected from a 4-7 membered partially unsaturated carbocyclic ring, phenyl, a 5-6 membered partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur;
   each R⁶ is independently -R, -C(O)R, -CO₂R, -C(O)C(O)R, -C(O)CH₂C(O)R, -S(O)R, -S(O)₂R, -C(O)N(R')₂, or -S(O)₂N(R')₂; or:
      R³ and R⁶ are taken together with their intervening atoms to form an optionally substituted fused ring selected from a 5-6 membered saturated or partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur.

Also described herein are methods which use a compound of Formula la, above, or a pharmaceutically acceptable salt thereof, in which:
R¹ is hydrogen or optionally substituted C₁₋₆ aliphatic;
X is -C(O)- or -S(O)₂-;
L¹ is a covalent bond or an optionally substituted C₁₋₄ alkylene;
A₁ is an optionally substituted bivalent ring selected from 3-7 membered saturated or partially unsaturated monocyclic carbocyclylene, 7-10 membered saturated or partially unsaturated bicyclic carbocyclylene, 4-7 membered saturated or partially unsaturated monocyclic heterocyclylene having 1-2 heteroatoms independently selected from nitrogen, oxygen, or sulfur, 7-10 membered saturated or partially unsaturated bicyclic heterocyclylene having 1-3 heteroatoms independently selected from nitrogen, oxygen, or sulfur, phenylene, 8-10 membered bicyclic arylene, 5-6 membered monocyclic heteroarylene having 1-3 heteroatoms independently selected from nitrogen, oxygen, or sulfur, or 8-10 membered bicyclic heteroarylene having 1-4 heteroatoms independently selected from nitrogen, oxygen, or sulfur;
L² is a covalent bond, or an optionally substituted alkylene chain;
Ring A₂ is a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered monocyclic heteroaryl ring having 1-3 heteroatoms independently selected from nitrogen, oxygen, or sulfur, an 8-10 membered bicyclic heteroaryl ring having 1-4 heteroatoms independently selected from nitrogen, oxygen, or sulfur, or a 10-16 membered saturated, partially unsaturated, or aromatic tricyclic ring having 0-4 heteroatoms independently selected from nitrogen, oxygen, or sulfur, wherein Ring A₂ is optionally substituted with 1-4 R^{x} groups;
each R^{x} is independently -R, optionally substituted alkylidenyl, oxo, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂;
each R is independently hydrogen or an optionally substituted group selected from C₁₋₆ aliphatic, a 3-7 membered saturated or partially unsaturated monocyclic carbocyclic ring, a 7-10 membered saturated or partially unsaturated bicyclic carbocyclic ring, a 4-7 membered saturated or partially unsaturated monocyclic heterocyclic ring having 1-2 heteroatoms independently selected from nitrogen, oxygen, or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur;
each R' is independently -R, or two R' groups on the same nitrogen are taken together with their intervening atoms to form an optionally substituted 5-8 membered saturated, partially unsaturated, or aromatic ring having 1-4 heteroatoms independently selected from nitrogen, oxygen, or sulfur;
L³ is a covalent bond or an optionally substituted C₁₋₄ alkylene chain;
   or L³ is unsubstituted methylene or methylene substituted with methyl or ethyl;
Ring A₃ is an optionally substituted ring selected from a 7-10 membered saturated or partially unsaturated bicyclic carbocyclic ring, a 4-7 membered saturated or partially unsaturated monocyclic heterocyclic ring having 1-2 heteroatoms independently selected from nitrogen,-or sulfur, a 7-10 membered saturated or partially unsaturated bicyclic heterocyclic ring having 1-3 heteroatoms independently selected from nitrogen, oxygen, or sulfur, a phenyl ring, an 8-10 membered bicyclic aryl ring, a 5-6 membered monocyclic heteroaryl ring having 1-3 heteroatoms independently selected from nitrogen, oxygen, or sulfur, or an 8-10 membered bicyclic heteroaryl ring having 1-4 heteroatoms independently selected from nitrogen, oxygen, or sulfur;
Ring A₄ is 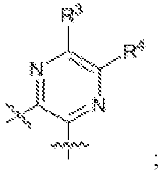 and
R³ is -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂;
R⁴ is -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -NHS(O)C₁₋₆alkyl, -N(R')S(O)₂N(R')₂, -N(R')S(O)₂R, or -OC(O)N(R')₂; or:
   R³ and R⁴ are taken together with their intervening atoms to form an optionally substituted fused ring selected from a 4-7 membered partially unsaturated carbocyclic ring, phenyl, a 5-6 membered partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur.

Such compounds and methods of their preparation are described in detail in international patent publication WO 2011-044157 A1.

Also described herein are methods of use of compounds of Formula I, in which Ring A3 is phenyl, substituted by one or two fluorines at the meta position or ortho position.

Also described herein are methods which use a compound of Formula Is: 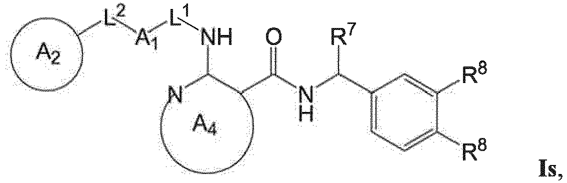 or a pharmaceutically acceptable salt thereof,
wherein each of A₁, A₂, L¹ and L² is as defined for Formula I, and
   any substitutable carbon on Ring A₄ is optionally substituted with R³, R⁴, or R⁵, and any substitutable nitrogen on Ring A₄ is optionally substituted with R⁶;
each of R³, R⁴, and R⁵ is independently -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂; or:
   R³ and R⁴ or R⁴ and R⁵ are taken together with their intervening atoms to form an optionally substituted fused ring selected from a 4-7 membered partially unsaturated carbocyclic ring, phenyl, a 5-6 membered partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur;
   each R⁶ is independently -R, -C(O)R, -CO₂R, -C(O)C(O)R, -C(O)CH₂C(O)R, -S(O)R, -S(O)₂R, -C(O)N(R')₂, or -S(O)₂N(R')₂; or:
      R³ and R⁶ are taken together with their intervening atoms to form an optionally substituted fused ring selected from a 5-6 membered saturated or partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur;
      R⁷ is hydrogen or methyl; and
      each R⁸ is independently hydrogen or halo.

Also described herein are methods which use a compound of Formula Iw: 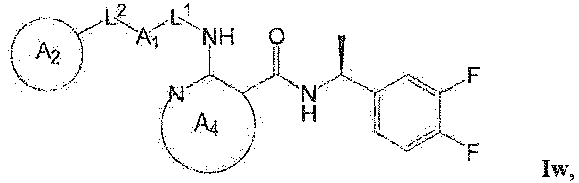 or a pharmaceutically acceptable salt thereof,
wherein each of A₁, A₂, L¹ and L² is as defined for Formula I, and any substitutable carbon on Ring A₄ is optionally substituted with R³, R⁴, or R⁵, and any substitutable nitrogen on Ring A₄ is optionally substituted with R⁶;
each of R³, R⁴, and R⁵ is independently -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂; or:
R³ and R⁴ or R⁴ and R⁵ are taken together with their intervening atoms to form an optionally substituted fused ring selected from a 4-7 membered partially unsaturated carbocyclic ring, phenyl, a 5-6 membered partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur;
each R⁶ is independently -R, -C(O)R, -CO₂R, -C(O)C(O)R, -C(O)CH₂C(O)R, -S(O)R, -S(O)₂R, -C(O)N(R')₂, or -S(O)₂N(R')₂; or:
   R³ and R⁶ are taken together with their intervening atoms to form an optionally substituted fused ring selected from a 5-6 membered saturated or partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur.

Also described herein are methods which use a compound of Formula Ix: 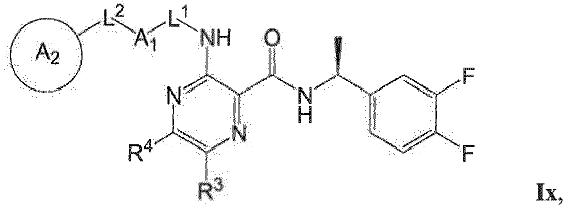 or a pharmaceutically acceptable salt thereof,
wherein each of A₁, A₂, L¹ and L² is as defined for Formula I, and each of R³ and R⁴ is independently -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂; or:
R³ and R⁴ are taken together with their intervening atoms to form an optionally substituted fused ring selected from a 4-7 membered partially unsaturated carbocyclic ring, phenyl, a 5-6 membered partially unsaturated heterocyclic ring having 1-3 heteroatoms independently selected from nitrogen, oxygen, or sulfur, or a 5-6 membered heteroaryl ring having 1-3 heteroatoms independently selected from nitrogen, oxygen, or sulfur.

Also described herein are methods which use a compound of Formula **Iy:** 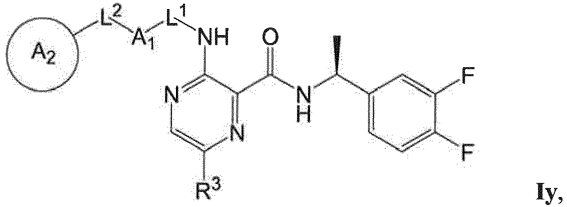 or a pharmaceutically acceptable salt thereof,
wherein each of A₁, A₂, L¹ and L² is as defined for Formula I, and R³ is -R, -halo, -NO₂, -CN, -OR, -SR, -N(R')₂, -C(O)R, -CO₂R, -C(O)C(O)R, -C(O)CH₂C(O)R, -S(O)R, -S(O)₂R, -C(O)N(R')₂, -S(O)₂N(R')₂, -OC(O)R, -N(R')C(O)R, -N(R')N(R')₂, -N(R')OR, -N(R')C(=NR')N(R')₂, -C(=NR')N(R')₂, -C=NOR, -N(R')C(O)N(R')₂, -N(R')S(O)₂N(R')₂, -N(R')S(O)₂R, or -OC(O)N(R')₂.

Also described herein are methods which use a compound of Formula **Iz:** 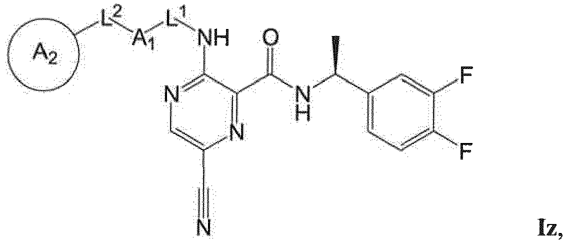 or a pharmaceutically acceptable salt thereof,
in which A₁, A₂, L¹ and L² are as defined for Formula I.

The methods may use any of the following compounds: 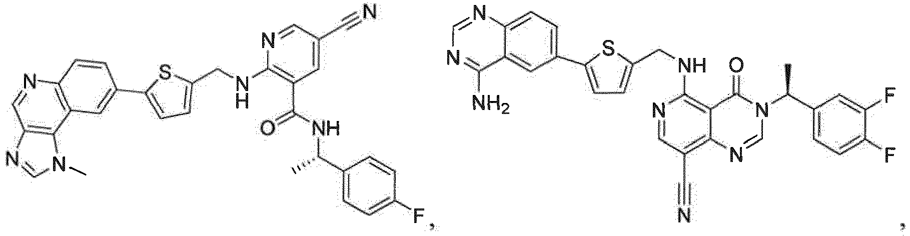 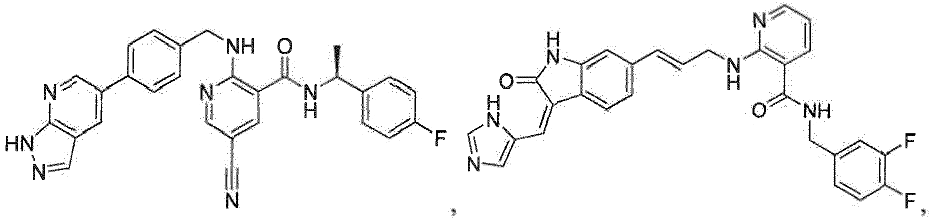 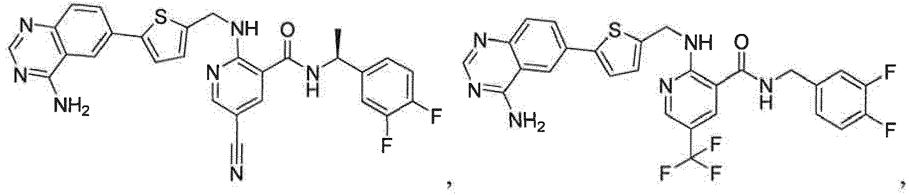 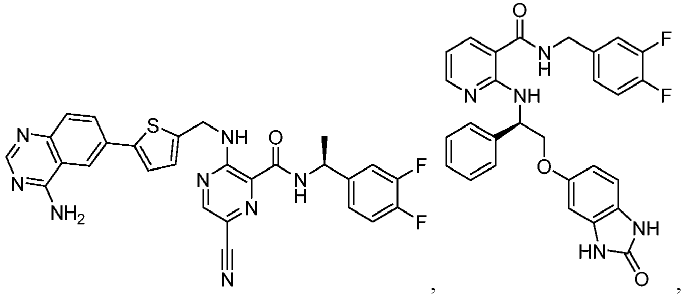 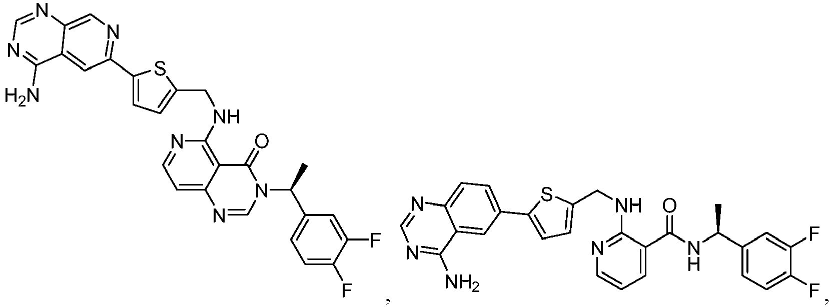 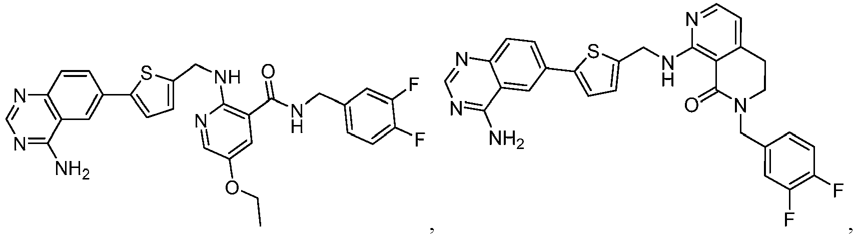 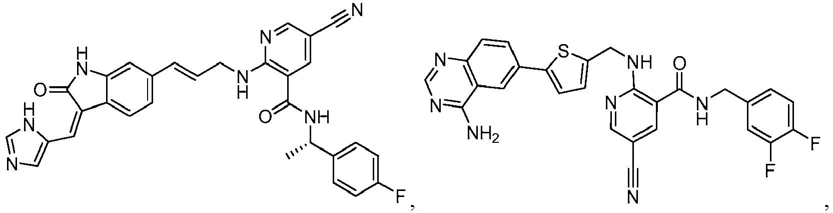 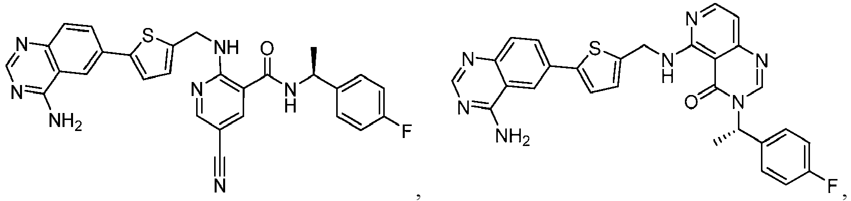 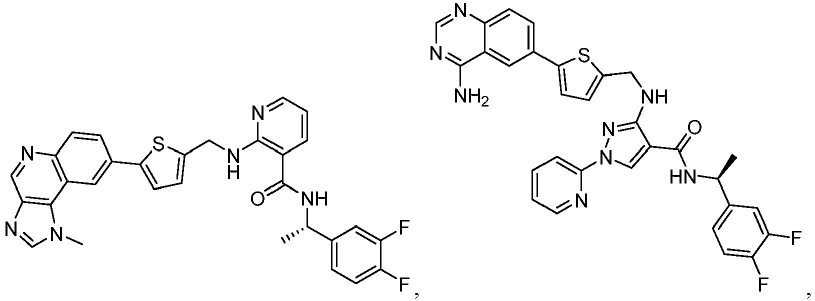 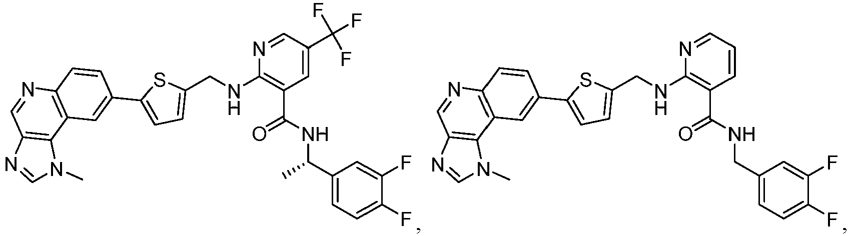 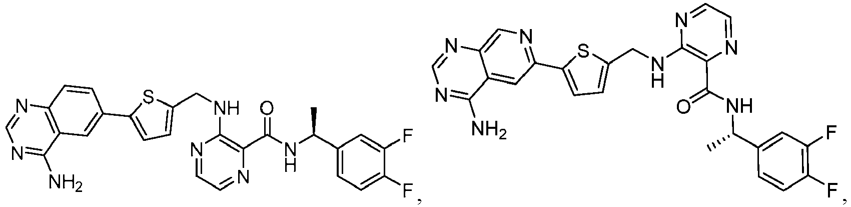 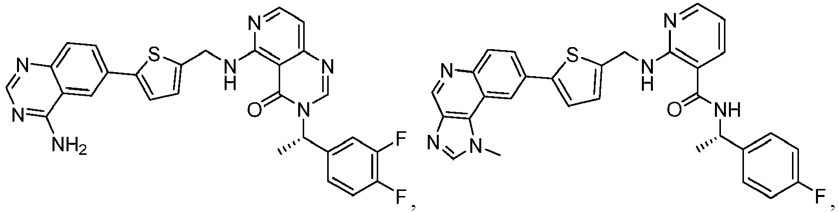 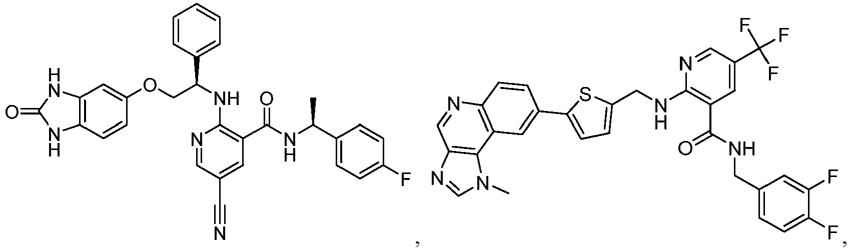 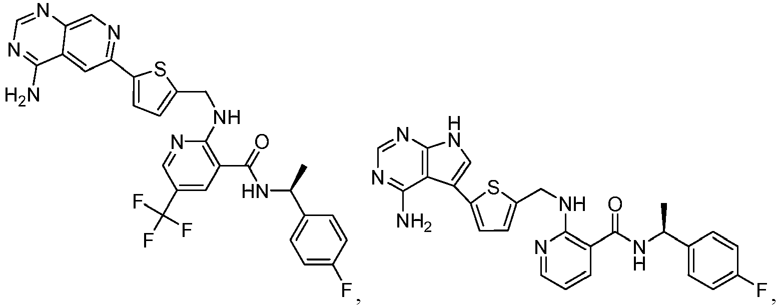 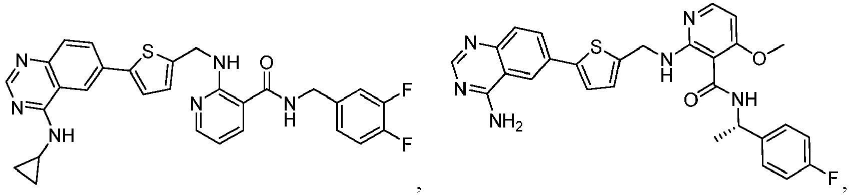 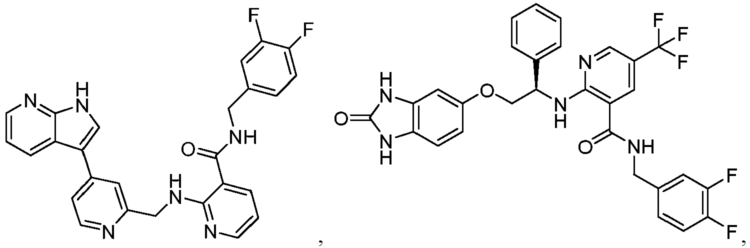 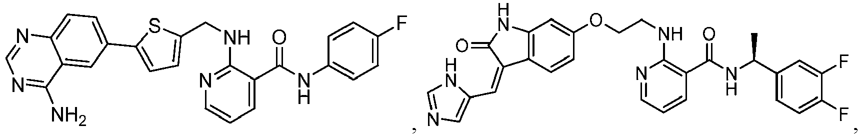 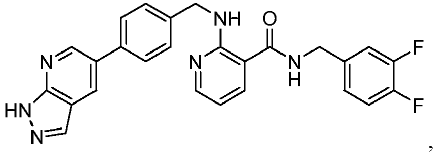 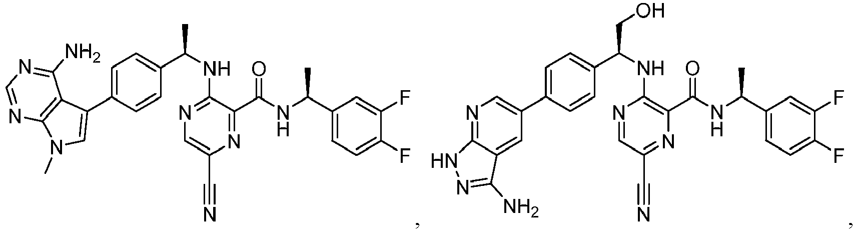 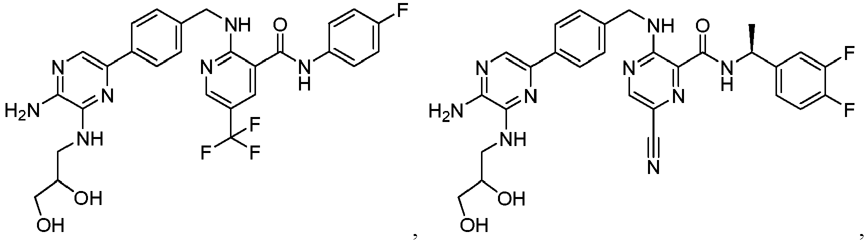 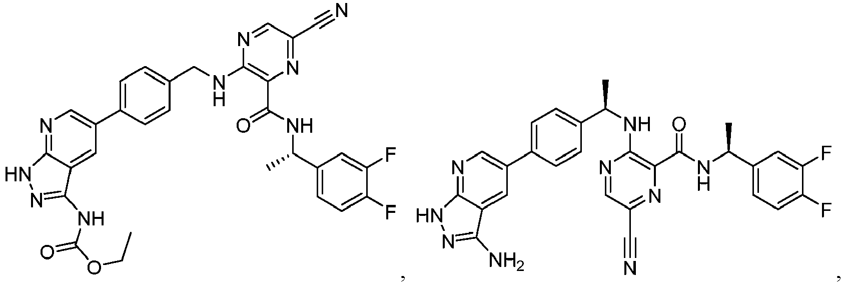 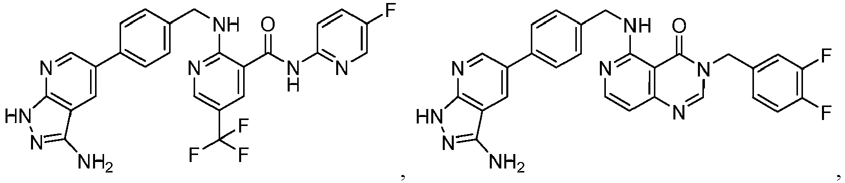 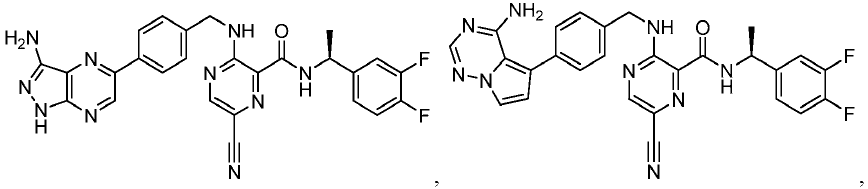 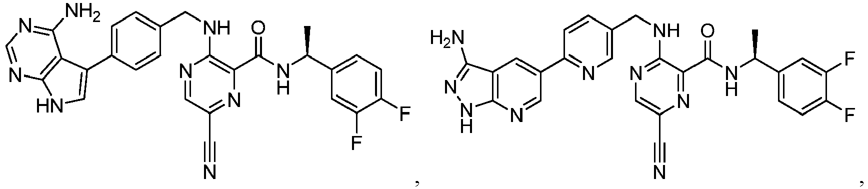 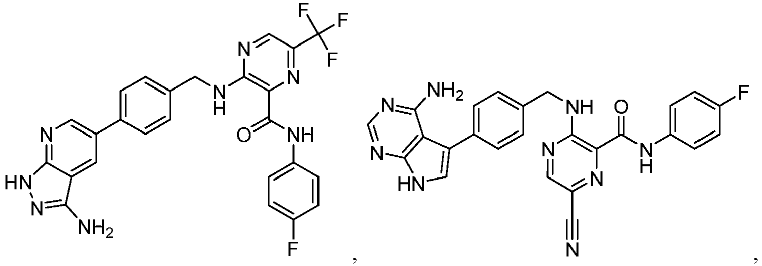 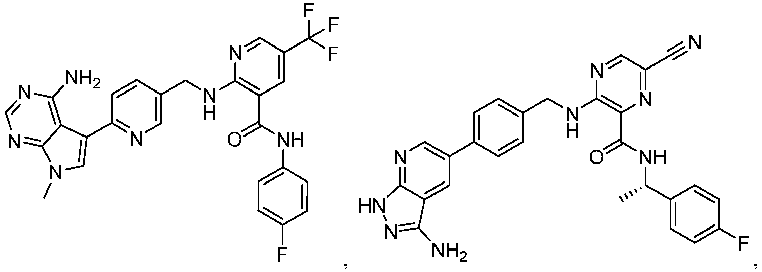 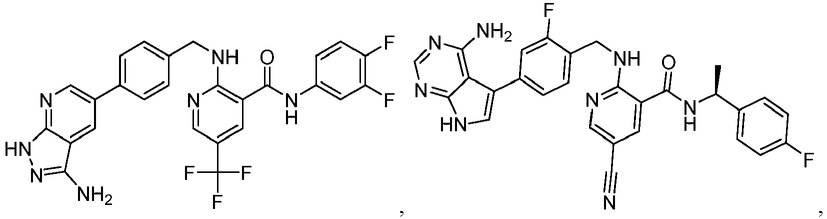 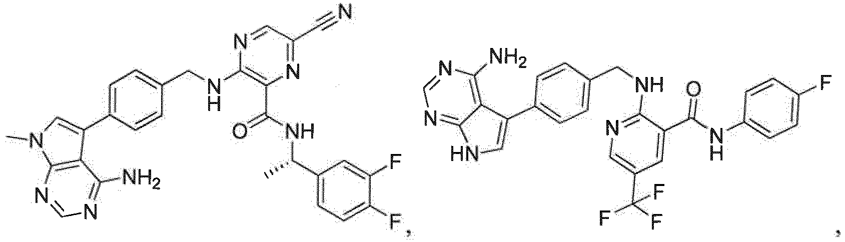 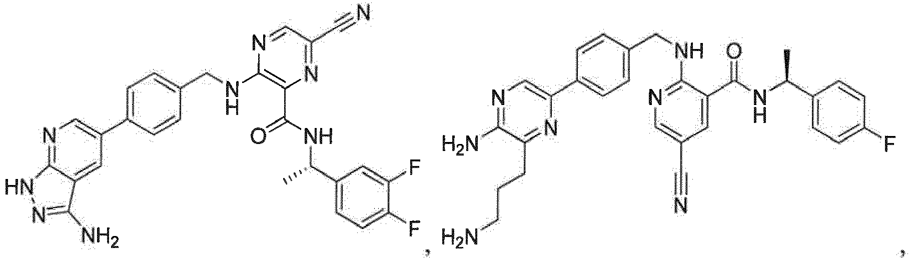 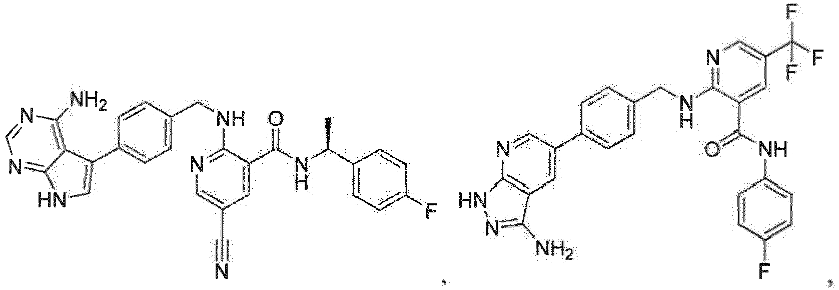 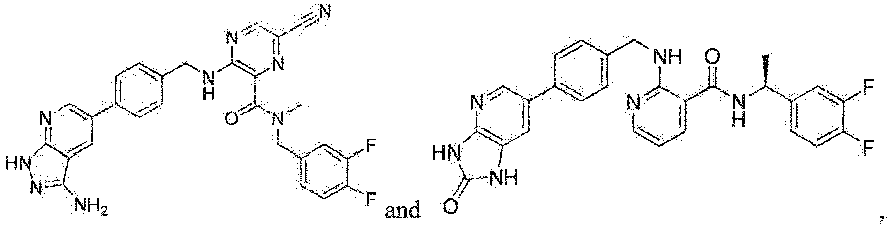 or a pharmaceutically acceptable salt thereof.

The present invention provides a compound selected from the group consisting of:

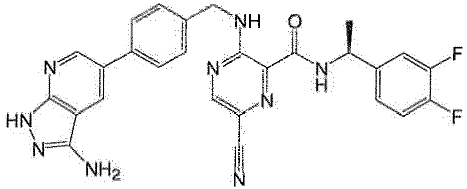

### 3-[4-(3-Amino-1H-pyrazolo[3,4-b]pyridin-5-yl)-benzylamino]-6-cyano-pyrazine-2-carboxylic acid [1-(3,4-difluoro-phenyl)-ethyl]-amide (Compound 3),

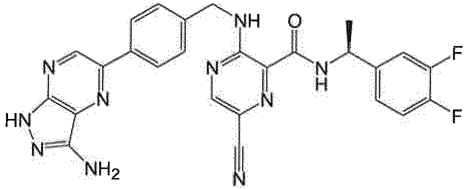

### 3-[4-(3-Amino-1H-pyrazolo[3,4-b]pyrazin-5-yl)-benzylamino]-6-cyano-pyrazine-2-carboxylic acid [1-(3,4-difluoro-phenyl)-ethyl]-amide(Compound 1),

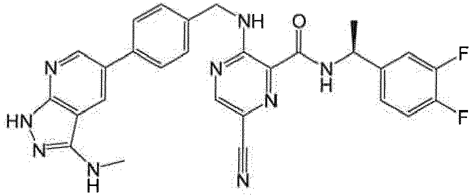

6-Cyano-3-[4-(3-methylamino-1H-pyrazolo[3,4-b]pyridin-5-yl)-benzylamino]-pyrazine-2-carboxylic acid [1-(3,4-difluoro-phenyl)-ethyl]-amide (Compound 2), or a pharmaceutically accpetable salt any of the foregoing, for use in a method of treating cancer in a subject in need thereof, in which cancer growth or survival is dependent on a PDK1-PIF-mediated substrate interaction, the method comprising administering to said subject a therapeutically effective amount of the compound or pharmaceutically acceptable salt thereof.

Also disclosed herein is a use of a compound of Formula I as described herein for the preparation of a medicament for the treatment of cancer in which PDK1-PIF-mediated substrate interaction-dependent cell survival pathways are implicated.

Also disclosed herein is a use of a compound of Formula I as described herein for the preparation of a medicament for the treatment of cancer in which RSK2-dependent cell survival pathways are implicated.

Also disclosed herein is a use of a compound of Formula I as described herein for the preparation of a medicament for the treatment of cancer in which Akt-independent cell survival pathways are implicated.

Definitions of specific functional groups and chemical terms are described in more detail below. For purposes of this invention, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Organic Chemistry, Thomas Sorrell, University Science Books, Sausalito, 1999; Smith and March March's Advanced Organic Chemistry, 5th Edition, John Wiley & Sons, Inc., New York, 2001; Larock, Comprehensive Organic Transformations, VCH Publishers, Inc., New York, 1989; Carruthers, Some Modern Methods of Organic Synthesis, 3rd Edition, Cambridge University Press, Cambridge, 1987.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention.

Where a particular enantiomer is preferred, it may, in some embodiments be provided substantially free of the corresponding enantiomer, and may also be referred to as "optically enriched." "Optically-enriched," as used herein, means that the compound is made up of a significantly greater proportion of one enantiomer. In certain embodiments the compound is made up of at least about 90% by weight of a preferred enantiomer. In other embodiments the compound is made up of at least about 95%, 98%, or 99% by weight of a preferred enantiomer. Preferred enantiomers may be isolated from racemic mixtures by any method known to those skilled in the art, including chiral high pressure liquid chromatography (HPLC) and the formation and crystallization of chiral salts or prepared by asymmetric syntheses. See, for example, Jacques et al., Enantiomers, Racemates and Resolutions (Wiley Interscience, New York, 1981); Wilen, et al., Tetrahedron 33:2725 (1977); Eliel, E.L., Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972).

The term "heteroatom" means one or more of oxygen, sulfur, nitrogen, phosphorus, or silicon (including, any oxidized form of nitrogen, sulfur, phosphorus, or silicon; the quaternized form of any basic nitrogen; or a substitutable nitrogen of a heterocyclic ring, for example N (as in 3,4-dihydro-2*H*-pyrrolyl), NH (as in pyrrolidinyl) or NR⁺ (as in N-substituted pyrrolidinyl)).

"PDK1 catalytic activity," as used herein, refers to PDK1 kinase catalytic activity. Thus, where PDK1 catalytic activity is decreased in the presence of a provided compound, the phosphorylation of a PDK1 substrate (e.g. Akt or PDK1 itself in the case of autophosphorylation) is decreased relative to the phosphorylation rate in the absence of the provided compound. In some embodiments, the IC₅₀ of a provided compound against PDK1 catalytic activity is less than 1 µM. In other embodiments, the IC₅₀ of a provided compound against PDK1 catalytic activity is less than 500 nM. In other embodiments, the IC₅₀ of a provided compound against PDK1 catalytic activity is less than 100 nM. In other embodiments, the IC₅₀ of a provided compound against PDK1 catalytic activity is less than 10 nM. In other embodiments, the IC₅₀ of a provided compound against PDK1 catalytic activity is less than 1 nM. In other embodiments, the IC₅₀ of a provided compound against PDK1 catalytic activity is from 0.1 nM to 10 µM. In other embodiments, the IC₅₀ of a provided compound against PDK1 catalytic activity is from 0.1 nM to 1 µM. In other embodiments, the IC₅₀ of a provided compound against PDK1 catalytic activity is from 0.1 nM to 100 nM. In other embodiments, the IC₅₀ of a provided compound against PDK1 catalytic activity is from 0.1 nM to 10 nM.

"PDK1 PIF-binding activity," as used herein, refers to PIF-dependent substrate binding by PDK1. Thus, where PDK1 PIF-binding activity is decreased in the presence of a provided compound, the phosphorylation of a PIF-binding-dependent PDK1 substrate (e.g., RSK2) is decreased relative to the phosphorylation rate in the absence of the provided compound. In some embodiments, the IC₅₀ of a provided compound against PDK1 PIF-binding activity is less than 1 µM. In other embodiments, the IC₅₀ of a provided compound against PDK1 PIF-binding activity is less than 500 nM. In other embodiments, the IC₅₀ of a provided compound against PDK1 PIF-binding activity is less than 100 nM. In other embodiments, the IC₅₀ of a provided compound against PDK1 PIF-binding activity is less than 10 nM. In other embodiments, the IC₅₀ of a provided compound against PDK1 PIF-binding activity is less than 1 nM. In other embodiments, the IC₅₀ of a provided compound against PDK1 PIF-binding activity is from 0.1 nM to 10 µM. In other embodiments, the IC₅₀ of a provided compound against PDK1 PIF-binding activity is from 0.1 nM to 1 µM. In other embodiments, the IC₅₀ of a provided compound against PDK1 PIF-binding activity is from 0.1 nM to 100 nM. In other embodiments, the IC₅₀ of a provided compound against PDK1 PIF-binding activity is from 0.1 nM to 10 nM.

"RSK2 activation activity," as used herein, refers to phosphorylation of RSK2, such as by PDK1. Thus, where RSK2 activation activity is decreased in the presence of a provided compound, the phosphorylation of RSK2 is decreased relative to the phosphorylation rate in the absence of the provided compound. In some embodiments, the IC₅₀ of a provided compound against RSK2 activation activity is less than 1 µM. In other embodiments, the IC₅₀ of a provided compound against RSK2 activation activity is less than 500 nM. In other embodiments, the IC₅₀ of a provided compound against RSK2 activation activity is less than 100 nM. In other embodiments, the IC₅₀ of a provided compound against RSK2 activation activity is less than 10 nM. In other embodiments, the IC₅₀ of a provided compound against RSK2 activation activity is less than 1 nM. In other embodiments, the IC₅₀ of a provided compound against RSK2 activation activity is from 0.1 nM to 10 µM. In other embodiments, the IC₅₀ of a provided compound against RSK2 activation activity is from 0.1 nM to 1 µM. In other embodiments, the IC₅₀ of a provided compound against RSK2 activation activity is from 0.1 nM to 100 nM. In other embodiments, the IC₅₀ of a provided compound against RSK2 activation activity is from 0.1 nM to 10 nM.

In another aspect, compounds of Formula I as described herein are useful for the treatment of one or more diseases, disorders, and/or conditions that may be alleviated by inhibiting (i.e. decreasing) certain PDK1 activities, including PI-independent PIF pocket substrate binding and PDK1-PIF mediated substrate interaction-dependent cell growth or proliferation. As used herein, the terms "treatment," "treat," and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

The present disclosure provides methods of treating cancer in a subject in need thereof. In some embodiments, provided methods include administering to the subject a therapeutically effective amount of a provided compound. The term "cancer" includes diseases or disorders involving abnormal cell growth and/or proliferation. In some embodiments, a cancer treated in accordance with the present invention is, by way of nonlimiting example, glioma, thyroid carcinoma, breast carcinoma, lung cancer (e.g., small-cell lung carcinoma, non-small-cell lung carcinoma), gastric carcinoma, cervical carcinoma, melanoma, skin carcinoma, colorectal carcinoma, gastrointestinal stromal tumors, pancreatic carcinoma, bile duct carcinoma, ovarian carcinoma, endometrial carcinoma, prostate carcinoma, renal cell carcinoma, anaplastic large-cell lymphoma, leukemia (e.g., acute myeloid leukemia, T-cell leukemia, chronic lymphocytic leukemia), multiple myeloma, malignant mesothelioma, malignant melanoma, colon cancer (e.g. microsatellite instability-high colorectal cancer).

In another aspect, cancers are hematologic cancers. In some embodiments, provided methods include administering to the subject a therapeutically effective amount of a provided compound. The term "hematologic cancer" includes blood-borne tumors and diseases or disorders involving abnormal cell growth and/or proliferation in tissues of hematopoietic origin, such as lymphomas, leukemias, and myelomas. Hematologic cancers that may be treated according to the invention include, by way of nonlimiting example, anaplastic large-cell lymphoma, non-Hodgkin's lymphoma, Hodgkin's lymphoma, B-cell lymphoma (e.g., ABC-diffuse large B-cell lymphoma, GCB-diffuse large B-cell lymphoma), T-cell lymphoma, mantle cell lymphoma, histiocytic lymphoma, T-cell leukemia, chronic lymphocytic leukemia, multiple myeloma, chronic myeloid leukemia, acute lymphocytic leukemia, acute myelogenous leukemia, and acute myeloblastic leukemia, plasma cell leukemia.

As used herein, the term "precancerous condition" means a condition, abnormal tissue growth, or lesion that tends or is likely to become cancerous. Precancerous conditions include, for example, actinic keratosis, adenomatous polyps of the colon, cervical dysplasia, and antecedent hematological disorders such as myelofibrosis, aplastic anemia, paroxysmal nocturnal hemoglobinuria, polycythemia vera, and myelodysplastic syndrome.

### Assays

To develop useful inhibitors of cancer growth, proliferation, or survival, candidate inhibitors capable of decreasing PDK1-PIF-mediated substrate interaction-dependent cell survival pathways may be identified *in vitro.* The activity of provided compounds can be assayed utilizing methods known in the art and/or those methods presented herein.

Compounds that decrease PDK1-PIF-mediated substrate interaction-dependent cell survival pathways may be identified and tested using biologically active PDK1 and other elements of these pathways, either recombinant or naturally-occurring. PDK1, RSK2, and Akt, for example, can be found in native cells, isolated *in vitro,* or co-expressed or expressed in a cell. Measuring the reduction in the PDK1-PIF-mediated substrate interaction-dependent cell survival pathways in the presence of an inhibitor relative to the activity in the absence of the inhibitor may be performed using a variety of methods known in the art, such as in the assays described herein. Other methods for assaying the activity of elements of PDK1-PIF-mediated substrate interaction-dependent cell survival pathways are known in the art. The selection of appropriate assay methods is well within the capabilities of those of skill in the art.

Compounds may be further tested in cell models or animal models for their ability to cause a detectable change in phenotype related to PDK1-PIF-mediated substrate interaction-dependent cell survival pathways. In addition to cell cultures, animal models may be used to test inhibitors of PDK1 for their ability to treat cancer in an animal model.

Compounds may be further tested for their ability to selectively inhibit or induce expression of genes or proteins that could serve as biomarkers to monitor inhibition of PDK1 activity in animal models or in healthy subjects or patients.

### Pharmaceutical Compositions

In another aspect, the present invention provides pharmaceutical compositions comprising a provided compound optionally in combination with a pharmaceutically acceptable excipient (e.g. carrier).

Provided pharmaceutical compositions include optical isomers, diastereomers, or pharmaceutically acceptable salts of the compounds disclosed herein. For example, in some embodiments, pharmaceutical compositions include a pharmaceutically acceptable salt. A compound included in the pharmaceutical composition may be covalently attached to a pharmaceutically acceptable carrier. Alternatively, the inventive compound included in the pharmaceutical composition is not covalently linked to a pharmaceutically acceptable carrier.

A "pharmaceutically acceptable carrier," as used herein refers to pharmaceutical excipients, for example, pharmaceutically, physiologically, acceptable organic, or inorganic carrier substances suitable for enteral or parenteral application which do not deleteriously react with the compounds used in accordance with the provided methods. Suitable pharmaceutically acceptable carriers include water, salt solutions (such as Ringer's solution), alcohols, oils, gelatins and carbohydrates such as lactose, amylose or starch, fatty acid esters, hydroxymethycellulose, and polyvinyl pyrrolidine. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like which do not deleteriously react with the compounds used in accordance with the provided methods.

Provided compounds can be administered alone or can be coadministered to a patient along with one or more other drugs. Coadministration is meant to include simultaneous or sequential administration of the compounds individually or in combination (more than one compound). In some embodiments, the preparations are combined with other active substances (e.g. to reduce metabolic degradation).

### Formulations

Compounds of the present invention can be prepared and administered in a wide variety of oral, parenteral, and topical dosage forms. In some embodiments, provided compounds are administered by injection (e.g. intravenously, intramuscularly, intracutaneously, subcutaneously, intraduodenally, or intraperitoneally). In some embodiments, compounds described herein are administered by inhalation, for example, intranasally. In some embodiments, provided compounds are administered transdermally. It is also envisioned that multiple routes of administration (e.g., intramuscular, oral, transdermal) can be used to administer the compounds of the invention. The present invention also provides pharmaceutical compositions comprising one or more provided compounds and one or more pharmaceutically acceptable carriers or excipients.

For preparing pharmaceutical compositions from provided compounds, pharmaceutically acceptable carriers can be either solid or liquid. Solid form preparations include powders, tablets, pills, capsules, cachets, suppositories, and dispersible granules. In some embodiments, a solid carrier is one or more substances, which may also act as diluents, flavoring agents, binders, preservatives, tablet disintegrating agents, or an encapsulating material.

In some embodiments, when the composition is a powder, the carrier is a finely divided solid in a mixture with the finely divided active component. In some embodiments, when the composition is formulated for a tablet, the active component is mixed with the carrier having the necessary binding properties in suitable proportions and compacted in the shape and size desired.

In some embodiments, provided powders and tablets contain from 5% to 70% of the active compound. Suitable carriers include magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. In some embodiments, the composition is formulated for a cachet or lozenge. In some embodiments, tablets, powders, capsules, pills, cachets, and/or lozenges are used as solid dosage forms suitable for oral administration.

In some embodiments, for preparing suppositories, a low melting wax, such as a mixture of fatty acid glycerides or cocoa butter, is first melted and the active component is dispersed homogeneously therein. The molten homogeneous mixture is then poured into convenient sized molds, allowed to cool and solidify.

Liquid form preparations include solutions, suspensions, and emulsions, for example, water or water/propylene glycol solutions. In some embodiments, for parenteral injection, liquid preparations can be formulated in solution in aqueous polyethylene glycol solution.

When parenteral application is needed or desired, particularly suitable admixtures for the compounds of the invention are injectable, sterile solutions, preferably oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, polyoxyethylene-block polymers, and the like. Ampules are convenient unit dosages. The compounds of the invention can also be incorporated into liposomes or administered via transdermal pumps or patches. Pharmaceutical admixtures suitable for use in the present invention include those described, for example, in Pharmaceutical Sciences (17th Ed., Mack Pub. Co., Easton, PA) and WO 96/05309.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations intended for conversion shortly before use to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

In some embodiments, provided pharmaceutical compositions are in unit dosage form. In such form the composition is subdivided into unit doses containing appropriate quantities of the active component. The unit dosage form can be a packaged preparation, the package containing discrete quantities of a pharmaceutical composition, such as packeted tablets, capsules, and powders in vials or ampoules. In some embodiments, the unit dosage form is a capsule, tablet, cachet, or lozenge itself, or it is the appropriate number of any of these in packaged form.

The quantity of active component in a unit dosage form may be varied or adjusted from 0.1 mg to 10000 mg, more typically 1.0 mg to 1000 mg, most typically 10 mg to 500 mg, according to the particular application and the potency of the active component. In some embodiments, provided compositions contain other compatible therapeutic agents.

Some compounds may have limited solubility in water and may require a surfactant or other appropriate co-solvent in the composition. Such co-solvents include: Polysorbate 20, 60 and 80, Pluronic F-68, F-84 and P-103, cyclodextrin, and polyoxyl 35 castor oil. Such co-solvents are typically employed at a level between about 0.01 % and about 2% by weight.

In some embodiments, viscosity greater than that of simple aqueous solutions may be desirable to decrease variability in dispensing the formulations, to decrease physical separation of components of a suspension or emulsion of formulation and/or otherwise to improve the formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose, chondroitin sulfate and salts thereof, hyaluronic acid and salts thereof, and combinations of the foregoing. Such agents are typically employed at a level between about 0.01% and about 2% by weight.

Provided compositions may additionally include components to provide sustained release and/or comfort. Such components include high molecular weight, anionic mucomimetic polymers, gelling polysaccharides and finely-divided drug carrier substrates. These components are discussed in greater detail in U.S. Pat. Nos. 4,911,920; 5,403,841; 5,212,162; and 4,861,760.

### Effective Dosages

Provided pharmaceutical compositions include compositions in which the active ingredient is contained in a therapeutically effective amount, *i.e.,* in an amount effective to achieve its intended purpose. The actual amount effective for a particular application will depend, *inter alia,* on the condition being treated. In certain embodiments, when administered in methods to treat cancer, provided compositions will contain an amount of active ingredient effective to achieve the desired result (e.g. decreasing the number of cancer cells in a subject).

The dosage and frequency (single or multiple doses) of administered to a mammal can vary depending upon a variety of factors, including a disease that results in increased activity of PDK1-PIF-mediated substrate interaction-dependent cell survival pathways, whether the mammal suffers from another disease, and its route of administration; size, age, sex, health, body weight, body mass index, and diet of the recipient; nature and extent of symptoms of the disease being treated (e.g., cancer), kind of concurrent treatment, complications from the disease being treated or other health-related problems. Other therapeutic regimens or agents can be used in conjunction with the methods and compounds of the invention.

For any compound described herein, a therapeutically effective amount may be initially determined from cell culture assays. Target concentrations will be those concentrations of active compound(s) that are capable of reducing the activity of PDK1-PIF-mediated substrate interaction-dependent cell survival pathways, as measured, for example, using the methods described herein.

Therapeutically effective amounts for use in humans may be determined from animal models. For example, a dose for humans can be formulated to achieve a concentration that has been found to be effective in animals. The dosage in humans can be adjusted by monitoring PDK1 inhibition and adjusting the dosage upwards or downwards, as described above.

Dosages may be varied depending upon the requirements of the patient and the compound being employed. In some embodiments, the dose administered to a patient is sufficient to effect a beneficial therapeutic response in the patient over time. The size of the dose also will be determined by the existence, nature, and extent of any adverse side-effects. In some embodiments, treatment is initiated with smaller dosages that are less than the optimum dose of the compound. Thereafter, the dosage is increased by small increments until the optimum effect under circumstances is reached. In one embodiment of the invention, the dosage range is 0.001% to 10% w/v. In another embodiment, the dosage range is 0.1% to 5% w/v.

### Combinations

Also disclosed herein are methods comprising administering a compound of Formula I or pharmaceutical compositions provided herein in combination with one or more second active agents, and/or in combination with radiation therapy or surgery.

The present invention provides a pharmaceutical composition for use in a combinational therapy of treating cancer in a subject, in which the growth or proliferation of the cancer is dependent on a PDK1-PIF-mediated substrate interaction comprising a formulation including a compound selected from the group consisting of: 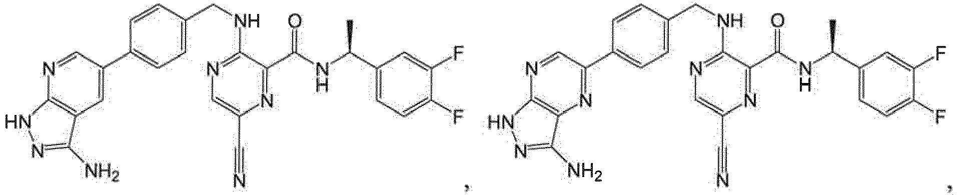 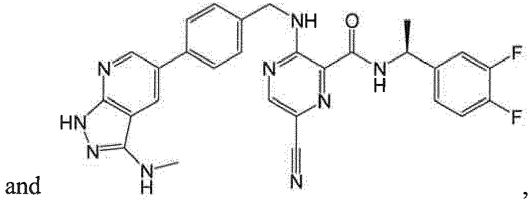 and pharmaceutically acceptable salts thereof; and a pharmaceutically acceptable carrier, wherein the combinational therapy further comprises an effective amount of a second anti-cancer agent.

Disclosed herein are therapies in which a patient may be administered an effective amount of a combination of a compound of Formula I and a second anti-cancer agent. In such combinational therapy, it is possible to administer amounts of each of the agents in the combination that are sub-therapeutic if such agents were to be administered alone, but that in combination the agents act in an additive or supra-additive manner to be therapeutically effective. However, some combinations may employ compounds in amounts that would otherwise be considered therapeutically effective by themselves, yet the combination proves to be more efficacious. In cancers, particularly, a standard of care may be altered by combination of agents, such that a treatment that is effective in some subset of patients becomes transformed into a new standard of care that is effective in a larger set of patients such as by prolonging life or by achieving a higher probability of remission.

Effective combinations of compounds of Formula I with other agents may be identified through preclinical and clinical testing of the combinations, and will depend on many factors, including disease type and stage of development, overall health of the patient, toxicities and side effects of the agents, and the like.

Examples of chemotherapeutic anticancer agents that may be used as second active agents in combination with of compound of Formula I include, but are not limited to, alkylating agents (e.g., mechlorethamine, chlorambucil, cyclophosphamide, melphalan, ifosfamide), antimetabolites (e.g., methotrexate), aurora kinase inhibitors (e.g., ZM447439, hesperidin, VX-680 AZD1152); purine antagonists and pyrimidine antagonists (e.g., 6-mercaptopurine, 5-fluorouracil (5-FU), cytarabine (Ara-C), gemcitabine), spindle poisons (e.g., vinblastine, vincristine, vinorelbine, paclitaxel), podophyllotoxins (e.g., etoposide, irinotecan, topotecan), antibiotics (e.g., doxorubicin, daunorubicin, bleomycin, mitomycin), nitrosoureas (e.g., carmustine, lomustine), inorganic ions (e.g., platinum complexes such as cisplatin, carboplatin), enzymes (e.g., asparaginase), hormones (e.g., tamoxifen, leuprolide, flutamide, and megestrol), topoisomerase II inhibitors or poisons, EGFR (Her1, ErbB-1) inhibitors (e.g., gefitinib), antibodies (e.g., bevacizumab, rituximab), IMIDs (e.g., thalidomide, lenalidomide), various targeted agents (e.g., HDAC inhibitors such as vorinostat), Bcl-2 inhibitors, VEGF inhibitors, proteasome inhibitors (e.g., bortezomib), cyclin-dependent kinase (cdk) inhibitors (e.g., seliciclib), quinolone derivatives (e.g., vosaroxin), and dexamethasone.

In other embodiments, compounds of Formula I may be used in combination therapy with PDK1 inhibitors, e.g., GSK2334470 (GlaxoSmithKline), BX-795, BX-912, and BX-320 (Berlex); Akt inhibitors, e.g., MK-2206 (Merck); PI3K inhibitors, e.g., GDC-0941 (pictilisib, Genentech), idelalisib (Gilead); BTK inhibitors,e.g., GS-4059 (Gilead).

In the treatment of hematological and solid tumors, second agents can include inhibitors of PD-1/PD-L1, for example, nivolumab (Opdivo), pembrolizumab (Keytruda, MK-3475), pidilizumab (CT-011), BMS 936559, and MPDL328OA; CTLA-4 inhibitors, for example, ipilimumab (Yervoy) and tremelimumab; and phosphatidylserine inhbitiors, for example, bavituximab (PGN401).

In the treatment of acute myelogenous leukemia, second agents include, for example, cytarabine (ara-C), daunorubicin, and vosaroxin.

In the treatment of CLL, second agents include, for example, PCI-32765 (ibrutinib).

In the treatment of myelomas, second agents include, for example, lenalidomide (Revlimid®) and bortezomib (Velcade®).

### EQUIVALENTS

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples that follow and the references to the scientific and patent literature cited herein.

It will be appreciated that for compound preparations described herein, when reverse phase HPLC is used to purify a compound, a compound may exist as a mono-, di-, or tri-trifluoroacetic acid salt.

It will further be appreciated that the present invention contemplates individual compounds described herein. Where individual compounds exemplified are isolated and/or characterized as a salt, for example, as a trifluoroacetic acid salt, the present invention contemplates a free base of the salt, as well as other pharmaceutically acceptable salts of the free base.

The following examples contain important additional information, exemplification and guidance that can be adapted to the practice of this invention.

### EXAMPLES

Without wishing to be bound by any particular theory, it is believed that compounds of Formula I bind the inactive conformation of PDK1 (IC₅₀ < 20 nM). The compounds bind deep in the adaptive (allosteric) pocket, causing a distortion in the N-terminal domain thereby perturbing the PIF-pocket and thus negatively modulating PI-independent substrate binding. Compound 2, for example, has been evaluated in a panel of more than 20 cell lines derived from hematologic cancers including acute myelogenous leukemia, multiple myeloma, DLBCL, and Mantle cell lymphoma, and shows strong anti-proliferative activity with EC₅₀ = 3-900 nM. Anti-proliferative activity correlated with pathway modulation assessed by inhibition of phosphorylation of PDK1, RSK2, and AKT. Interestingly, inhibition of PDK1 phosphorylation was time-dependent, showing 2-5-fold more inhibition after 24 hours than 4 hours. In addition, Compound 2 produced substantial apoptosis after 24 hours. Compound 2 was compared to the PDK1 inhibitor GSK2334470, showing comparable biochemical potency, but Compound 2 was 10- to 30-fold more potent at inhibiting PDK1 and RSK2 phosphorylation in all cell lines tested. In addition, Compound 2 was at least 10-fold more potent than GSK2334470 in 72 hours viability assays.

In mice, Compound 1 and Compound 2 are orally bioavailable (%F > 40%) with a Tₘₐₓ of 4-8 hours and long half-life. Pathway modulation was assessed in vivo using MV4-11 xenografts in mice. Potent pathway modulation was observed at 4 hours and 24 hours after a single oral dose of Compound 1 and Compound 2. Efficacy was assessed by 21-day dosing in MV4-11 xenografts. Both Compound 1 and Compound 2 show dose-related efficacy with TGI reaching 96-97% and partial regression in 70-100% of animals at the highest dose.

Without wishing to be bound by any particular theory, it is believed that targeting the inactive conformation of PDK1 and inhibiting PI-independent substrate binding has broad potential for the treatment of solid and hematologic cancers, especially in contexts in which PDK1 kinase inhibitors or Akt inhibitors are insufficiently effective.

### Example 1 - PDK1 kinase activity assay

Full-length PDK1 protein (SignalChem) was dephosphorylated using GST-λ-phosphatase (produced in house), which was subsequently removed using glutathione-agarose beads (Gold Biotechnology). Full-length AKT Ser476Asp (5 nM) was incubated with PDK1 (40 pM phosphorylated or 100 pM unphosphorylated), 100 nM FITC-Crosstide (GSK-3 Ser 21 peptide, CGSGSGRPRTSSFAEG (SEQ ID NO.: 1); ThermoFisher) and 24 µM ATP for 2 hr in 10 mM Tris (pH 7.5) containing 10 mM MgCl₂, 0.01% Triton X-100, and 1 mM dithiothreitol (DTT), in the presence or absence of test compounds, which were added using an Echo 555 acoustic dispenser (Labcyte). Tb-pCrosstide antibody (ThermoFisher) was then added to a final concentration of 2 nM and the reaction was incubated for an additional 30 min. Fluorescence resonance energy transfer (FRET) was measured using a Tecan Infinite F500 plate reader with λₑₓ = 340 nm, λₑₘ₁ = 485 nm, λₑₘ₂ = 520 nm.

Figures 2A and 2B shows PDK1 kinase activity inhibition curves in this assay for Compound 1 and Compound 2. Inset values in each graph provide the IC₅₀ values. These data confirm that representative compounds of Formula I are potent inhibitors of both phosphorylated and un-phosphorylated PDK1.

### Example 2 - Selectivity

The selectivity of Compound 1 and Compound 2 was evaluated in a panel of 270 different human kinases offered by Upstate (now Millipore). The compounds were tested at 10 µM concentration and showed inhibition greater than or equal to 90% for 20 kinases (including PDK1) for Compound 2 and 18 kinases (including PDK1) for Compound 1, demonstrating great selectivity (inhibition of less than 10% of kinome) at this high concentration (greater than 1,000-fold the IC₅₀ concentration for PDK1).

### Example 3 - Cell Proliferation Assay (MTS)

Cell proliferation was measured using the CellTiter 96 Aqueous NonRadioactive Cell Proliferation Assay (Promega). The CellTiter 96® AQueous Assay is composed of solutions of a tetrazolium compound [3-(4,5-dimethylthiazol-2-yl)-5-(3-carboxymethoxyphenyl)-2-(4-sulfophenyl)-2H-tetrazolium, inner salt; MTS] and an electron coupling reagent (phenazine methosulfate) PMS. MTS is bioreduced by cells into a formazan product that is soluble in tissue culture medium. The absorbance of the formazan product at 490 nm can be measured directly from assay plates without additional processing. The quantity of formazan product as measured by the amount of 490 nm absorbance is directly proportional to the number of living cells in culture. Cells were seeded into 96-well or 384-well clear plates in a volume of 200 µL (96-well) or 50 µL (384-well) at optimized densities ranging from 1,000 to 40,000 cells per well for 96-well plates or 1,200 to 25,000 cells per well for 384-well plates depending on cell-line. After 4-6 hr recovery, serially diluted compounds in DMSO were added to the cells using (0.1% v/v final DMSO concentration). Cells were then grown at 37 °C in a humidified incubator with 5% CO₂ for 72 hr. A 20:1 [3-(4,5-dimethylthiazol-2-yl)-5-(3-carboxymethoxyphenyl)-2-(4-sulfophenyl)-2H-tetrazolium] (MTS):phenazine methosulfate (PMS) solution in DPBS was made immediately before use, added to each well, and cells were incubated for 1.5 hr at 37 °C. The absorbance at 490 nm was then measured using a platereader. Absorbance values were normalized as percent of control (cells incubated in the presence of 0.1% v/v DMSO). Sigmoidal dose-response curves were plotted using log(inhibitor) vs. response - Variable slope (four parameters) model with a top value constrained to 100% (GraphPad Prism version 6.00 for Windows, GraphPad Software, La Jolla California USA). Bottom value was constrained to 0% for compounds that did not reach a plateau at higher concentration range. Curves for compounds that reached a plateau were matched exactly to the measurement points. For curves that reach a high plateau (>20%), EC₅₀ is reported as the concentration that results in 50% inhibition of cell growth. To determine potential of compounds of the invention to affect viability of hematological cancers, cell lines representing various tumor types were selected and tested. Human cell lines tested were: MOLM-13 (acute myeloid leukemia (AML)), MV4-11 (AML), U-2932 (ABC-diffuse large B cell lymphoma), U-937 (histiocytic lymphoma), U-266 (multiple myeloma), RPMI-8226 (multiple myeloma), CMK (megakaryoblastic cell line), SU-DHL-4 (GCB-diffuse large B cell lymphoma), KG1 (AML), Mec-1 (chronic B-cell leukemia), MOLM-16 (AML), Jeko (B-cell lymphoma), WSU-DLCL2 (B-cell lymphoma), JJN3 (plasma cell leukemia), SU-DHL-6 (GCB-diffuse large B cell lymphoma), and Z-138 (mantle cell lymphoma). In addition, two murine cell lines were tested: A20 (AML) and C1498 (ABC-diffuse large B cell lymphoma). Compound 3, Compound 1, Compound 2, GSK-2334470 (PDK1 inhibitor, GlaxoSmithKline, Figures 12A-12E), MK-2206 (AKT inhibitor; Merck), and GDC-0941 (pictilisib; pan-PI3K inhibitor; Genentech) were tested for effect in this assay. The representative compounds Compound 3, Compound 1, and Compound 2 demonstrated potent inhibition of cell proliferation with EC₅₀ between 3 nM and 853 nM against the various cell lines, indicating broad effect across tumor types (Table 1).

**Table 1**

| **MTS proliferation assay** (µM) | Compound 1 | Compound 2 | Compound 3 |
|---|---|---|---|
| Molm-13 | 0.006 | 0.003 | 0.005 |
| MV4-11 | 0.005 | 0.007 | 0.012 |
| U-2932 | 0.108 | 0.056 | 0.115 |
| U-937 | 0.123 | 0.074 | 0.111 |
| U-266 | 0.226 | 0.130 | 0.145 |
| RPMI-8226 | 0.174 | 0.163 | 0.201 |
| CMK | 0.222 | 0.182 | 0.278 |
| SU-DHL-4 | 0.313 | 0.186 | 0.411 |
| KG1 | 0.175 | 0.195 | 0.193 |
| Mec-1 | 0.415 | 0.215 | 0.403 |
| MOLM-16 | 0.248 | 0.227 | 0.291 |
| Jeko | 0.286 | 0.279 | 0.249 |
| WSU-DLCL2 | 0.543 | 0.354 | 0.474 |
| JJN3 | 0.846 | 0.453 | 0.853 |
| SU-DHL-6 | 0.572 | 0.476 | 0.458 |
| Z-138 | 0.718 | 0.684 | 0.754 |

As shown in Figure 3, the MK-2206 and GDC-0941 compounds showed substantial variability in potency in the cell proliferation assay with activities ranging from 0.1 µM to 10.7 µM (EC₅₀ > 1 µM for 9 out of 16 cell lines) for MK-2206 and ranging from 0.05 µM to 6.1 µM (EC₅₀ > 1 µM for 3 out of 16 cell lines) for GDC-0941. This is in contrast to the potencies observed for Compound 2, which shows growth inhibition of all cell lines at concentrations less than 1 µM and in most cases at concentrations less than 500 nM (15 out of 16 cell lines). These data suggest that the compounds of the invention may be useful in a variety of tumors that are less susceptible to inhibitors that target other kinases in the same pathway, potentially being useful as first line therapeutics, or as rescue therapeutics in cases where other kinase inhibitors are or become ineffective treatments.

### Example 4 - Cell Proliferation Assay (MTS)

For a subset of cell lines, representative Formula I compounds were compared to GSK2334470 and investigated in more detail. Table 2 shows comparative EC₅₀ data for the various compounds for inhibition of cell proliferation in seven cell lines. In most cases (6 out of 7 cell lines), the test compound ranges from 7- to 50-fold more potent than GSK2334470.

**Table 2**

| **MTS proliferation assay** (µM) | Compound 1 | Compound 2 | **MK-2206** | **GSK-2334470** |
|---|---|---|---|---|
| Molm-13 | 0.006 | 0.003 | 1.369 | 0.360 |
| MV4-11 | 0.005 | 0.007 | 1.300 | 0.105 |
| U-2932 | 0.108 | 0.056 | 4.880 | 0.986 |
| RPMI-8226 | 0.174 | 0.163 | 0.906 | 2.900 |
| Jeko | 0.286 | 0.279 | 0.980 | 2.250 |
| A20 | 0.156 | 0.114 | 0.138 | 0.49 |
| C1498 | 0.053 | 0.045 | 0.812 | 0.358 |

Further examination of the growth inhibition curves show that the test compounds produce low plateaus (<20%, as observed for doxorubicin, a widely used and efficacious anti-cancer drug) at higher concentrations indicative a cell killing (fewer cell remaining after 72 hrs than was plated at the start of the experiment) probably by induction of apoptosis (Figures 4A-4D). In contrast, the curves for GSK2334470 and MK-2206 do not reach a plateau below 20% for MV4-11 and C1498, suggesting that these compounds partially inhibit cell growth but do not induce cell death. Effective cancer drugs induce growth inhibition indicative of cell death (as illustrated by doxorubicin in Figures 4A-4D and 5A-5C). These data suggest that the compounds of Formula I may have utility as anti-cancer agents.

### Example 5 - Induction of apoptosis

Induction of apoptosis was assessed using an Alexa Fluor 488 Annexin 5/Dead Cell Apoptosis kit for flow cytometry (Life Technologies) essentially per the manufacturer's instructions. Cells were seeded in a six-well tissue culture plate in 3 mL complete growth medium (3 x 10⁵ cells/well) and allowed to equilibrate for 1 hr at 37 °C in a humidified incubator with 5% CO₂. DMSO (control), test compound, or doxorubicin was added to the wells (0.1% final DMSO concentration). Cells were returned to the incubator for 24 hrs. On the day of analysis 1 x 10⁵ cells were labeled according to manufacturer's instructions and analyzed by flow cytometry (BD FACSCalibur™, GH) to determine the percent of apoptotic cells (annexin V positive, AV+) and/or apoptotic dead cells (AV+ and propidium iodide positive (PI+)).

This example shows that Compound 1 and Compound 2 are efficient at inducing apoptosis at concentrations as low as 50 nM, which is consistent with the growth inhibition data of Figure 3 that show a plateau well below 20% in 50-100 nM range. In contrast, GSK-2334470 does not show signs of apoptosis induction even at concentrations as high as 300 nM.

### Example 6 - Western Blot Analysis

For phosphoprotein analyses, cells were seeded into 10 cm petri dishes (10 x 10⁶ cells/dish) in media supplemented with 10% FBS and penicillin/streptomycin. After cells recovered for 1 hr at 37 °C, compounds were added in DMSO (0.1% final) and incubated for 4 or 24 hr at 37 °C. Cells were then harvested by centrifugation and washed with cold PBS. Cell pellets were resuspended in cell extraction buffer (Invitrogen) supplemented with 2X Halt™ protease and phosphatase inhibitor cocktail (Thermo Scientific), 2 mM sodium orthovanadate, 10 mM EDTA and 4 mM PMSF. Samples were lysed by sonication and incubated on ice for 30 min. Cell debris was removed by centrifugation and protein concentrations were determined using the BCA Protein Assay Kit (Pierce). The clarified lysate was diluted in LDS sample buffer with reducing agent (Life Technologies). After heating to 70 °C samples were cooled and loaded onto 4-12% Bis-Tris gels (Life Technologies) at 50 µg/well. Gels were run at 110 V and proteins were transferred to a PVDF membrane. Membranes were blocked in TBS blocking buffer (Li-Cor Biosciences) for 1 hr. Primary antibodies (Cell Signaling, Sant Cruz Biotechnology) were added (1:1,000 dilution) in blocking buffer with 0.1% Tween-20 and 1:20,000 dilution of β-actin antibody and incubated for 14 hr at 4 °C. Blots were washed in TBS-T. Label-conjugated goat anti-rabbit secondary antibody (IRdye® 800CW; Li-Cor Biosciences) was added in blocking buffer with 0.1% Tween-20 and 0.02% SDS and incubated for 1 hr. After washing in TBS-T followed by TBS, membranes were scanned on an Odyssey imaging system (Li-Cor Biosciences). Bands were quantitated using ImageJ.

Figure 6A shows that Compound 2 and GSK-2334470 each modulate PDK1 and RSK2 phosphorylation. Figures 6C shows that Compound 2 appears to be 10-fold more potent than GSK-2334470 in inhibiting phosphorylation of both PDK1 and RSK2 (see dotted line in Figure 5B). Figure 6C shows that Compound 2 (30 nM) inhibition of RSK2 phosphorylation appears to be potent and time-independent, whereas and GSK2334470 at the same concentration has little or no effect on RSK2 phosphorylation. By contrast, inhibition of PDK1 phosphorylation is time-dependent for both compounds, with Compound 2 showing a 5-fold decrease in PDK1 phosphorylation between 4 hr and 24 hr exposure, suggesting that PDK1 dephosphorylation is a slow process that requires prolonged PDK1 occupancy. It should be noted that PDK1 is activated by autophosphorylation and thus, once PDK1 becomes unphosphorylated while inhibitor is still present, the pathway will be completely shut down. At 100 nM concentration, Compound 2 achieves 90% inhibition of P-PDK1 and >95% inhibition of P-RSK2, while GSK2334470 only achieves 70% and 55% inhibition, respectively, at the same concentration. This discrepancy may explain why Compound 2 induces apoptosis at 100 nM while GSK2334470 only achieves partial growth inhibition with no evidence of apoptosis. In data not shown, only weak AKT-T308 signal was observed. Note that the MK-2206 Akt inhibitor was not effective in the MTS cell proliferation assay described in Example 4 above, suggesting that PDK1-mediated survival in this cell line primarily goes through RSK2. This is consistent with literature data showing that the RSK2 siRNA inhibits growth of MV4-11 cells and triggers apoptosis (Elf et al., Blood, 2011, 117(25):6885-6894).

Figures 7A-C show that the C1498 B-cell lymphoma cell line (ABC-type) is sensitive to Compound 2, showing >90% P-RSK2 and ∼80% P-PDK1 inhibition in the 30-100 nM range. The effect of GSK2334470 is much weaker reaching approximately 75% inhibition of both P-PDK1 and P-RSK2 at 300 nM. Correspondingly, Compound 2 shows 80% growth inhibition at 200 nM, while GSK2334470 does not achieve this effect until a concentration of 5-10 µM. Like MV4-11, C1498 does not show significant AKT or IKK phosphorylation, suggesting that PDK1 mediated survival primarily goes through RSK2. Interestingly, treatment with the AKT inhibitor MK-2206 appears to enhance PDK1 and RSK2 phosphorylation.

Figures 8A-8E show that the A20 AML cell is less sensitive to Compound 2, in that 300 nM compound is required to achieve >90% inhibition of P-RSK2 and that P-PDK plateaus at 70% inhibition. This is consistent with substantially higher EC₅₀s in the proliferation assay as compared to MV4-11 and C1498. A20 cells are even less sensitive to GSK2334470 showing only 45% P-PDK1 inhibition at the highest concentration. In contrast to previous cell lines, A20 show AKT activation, and P-AKT levels are sensitive to the AKT inhibitor MK-2206. This is consistent with potent growth inhibition by MK-2206 as shown in Figure 3. P-AKT levels are also sensitive to Compound 2 while being barely affected by GSK2334470. These data show a clear correlation between pathway modulation by Compound 2 and MK-2206 and inhibition of cell growth, while GSK2334470 only shows a moderate effect in the two assays.

Figures 9A-C show that the KG-1 cell line is sensitive to Compound 1, with >80% inhibition of pRSK2 and pPDK1 levels at 100 nM compound. Like the C1498 cell line, KG-1 cells do not show significant AKT phosphorylation.

Taken together, the data indicate that 70-80% P-PDK1 inhibition and >90% P-RSK2 inhibition may be required to effectively inhibit cell proliferation and potentially trigger apoptosis. Importantly, growth inhibition and apoptosis does not require inhibition of AKT and IKK in several cell lines, instead pointing to P-RSK2 as a key driver of cell survival. This is an unexpected finding, but highlights the important anti-cancer potential of the compounds of Formula I. While GSK2334470 can clearly inhibit P-PDK1 in cells, the compound is typically 10-30 fold less potent in the pathway modulation and growth inhibition assays when compared to Compound 2. The same is true for P-RSK2 inhibition which reaches 65-85% at the highest concentration tested. This may explain why GSK2334470 has failed to show efficacy in animal studies and has not been advanced as a drug candidate.

Compound 1 was also assessed in the above assays, providing results similar to those described for Compound 2.

### Example 7 - Pathway modulation in tumor xenografts

MV4-11 cells propagated in vitro were implanted subcutaneous into the right flank of 9-week old female NCr nu/nu mice. On the day of implant, MV4-11 cells were harvested during log phase growth and resuspended in phosphate buffered saline (PBS) containing 50% Matrigel™ (BD Biosciences) at a concentration of 1 x 10⁸ cells/mL. Xenografts were initiated by subcutaneously implanting 1 x 10⁷ MV-4-11 cells (0.1 mL suspension) into the right flank of each test animal and tumors were monitored as their volumes approached the target range of 175 to 225 mm³. Two weeks after implantation, animals were assigned to individual groups of 3 animals with and average tumor volume of approximately 200 mm³. Animals were dosed by oral gavage with 5 mL/kg vehicle (1% DMA / 99% Labrasol), Compound 1 (in 1% DMA / 99% Labrasol), Compound 2 (in 1% DMA / 99% Labrasol), GDC0941 (in 0.5% methylcellulose : 0.2% Tween 80 in DI Water), and by intraperitoneal injection (10 mL/kg) of GSK2334470 (in 1% DMSO, 20% PEG400, pH 4.2). Actual dosing concentrations are shown in Figures 10A-10D. Eight hours post-dose, full blood volume was collected by cardiac puncture under isoflurane anesthesia and tumors were excised and snapfrozen in liquid nitrogen and stored at -80 °C until further analysis. Tumors were pulverized in liquid nitrogen using a Biopulverizer and split into two sample tubes: one for Western blot analysis and one for analysis of compound levels by LC-MS/MS. Western blot analysis was performed as described above. For MS analysis, tumor samples were homogenized with a Virsonic 100 ultrasonic homogenizer. Each sample was first weighed, and then an appropriate volume of 20:80 methanol:water was added to make a 9 mL/gram sample. Samples were then homogenized on ice, and stored frozen until analysis. Standards were prepared in BALB/c mouse plasma or blank homogenized tumor tissue. Standards and samples were analyzed on a PE Sciex API4000 instrument and compound concentration was quantified and back-calculated to ng compound per g tumor tissue and converted to µM concentration assuming 1 g tumor tissue equals 1 mL volume.

MV4-11 tumors were grafted onto mice to assess the utility of representative compounds of Formula I as modulators of PDK1 signaling in tumors in vivo. As shown in Figures 10A and 10B, Compound 1 and Compound 2 inhibit PDK1 in MV4-11 tumors 4 hrs and 8 hrs after a single oral gavage of compound. The inhibition is dose-dependent and stronger at 8 hrs reaching 50-60% inhibition of PDK1 phosphorylation at the highest concentration and mirrors the time dependent inhibition of P-PDK1 observed in cells in vitro. The P-PDK1 inhibition results in strong suppression of RSK2 and AKT phosphorylation with up to 80-90% inhibition 8 hrs post dose (Figures 10C-D). Compound 1 and Compound 2 concentrations in tumors roughly correspond to the doses given. The PI3K inhibitor GDC0941 produces 45% inhibition of PDK1 at 8 hrs, but only a modest 25% inhibition of P-PDK1 and no inhibition of P-AKT. Despite the high dose of 50 mg/kg GDC0941, tumor exposure was only 0.4-1.4 mM, comparable to the exposure achieved with the 1 mg/kg dose of Compound 1 and Compound 2. In agreement with this observation, the pathway modulation effects observed for 50 mg/kg GDC0941 were comparable to those of 1 mg/kg Compound 1 or Compound 2. The PDK1 inhibitor GSK2334470 dosed IP at 50 mg/kg showed pathway modulation and tumor exposure comparable to that of 50 mg/kg GDC0941 and 1 mg/kg Compound 1 or Compound 2.

The data from these experiments show that the compounds of Formula I are capable of producing strong PDK1 pathway modulation in tumors in vivo at doses that are physiological relevant for treating human cancers, thus underscoring the utility of compounds of the invention as potential therapies for human cancer. Furthermore, the effects of compounds of Formula I compares very favorably with other compounds targeting PDK1 activity or the PI3K pathway.

### Example 8 - Tumor xenograft efficacy

MV4-11 cells were propagated in vitro and implanted subcutaneous into the right flank of 9-week old female NCr nu/nu mice as described in Example 7. The animals were fed ad libitum water (reverse osmosis, 1 ppm Cl), and NIH 31 Modified and Irradiated Lab Diet® consisting of 18.0% crude protein, 5.0% crude fat, and 5.0% crude fiber. Fourteen days after tumor implantation, designated as Day 1 of the study, the animals were sorted into seven groups each consisting of ten mice with individual tumor volumes of 108 to 288 mm³ and group mean tumor volumes (MTV) of 162 to 165 mm³. On Day 1 of the study, dosing by oral gavage was initiated as follows: The dosing volume was 0.100 mL per 20 grams of body weight (5 mL/kg), and was scaled to the body weight of each individual animal. Group 1 mice received vehicle and served as the control group. Groups 2-4 received Compound 1 at 5, 11, and 25 mg/kg, respectively, qd x 21. Groups 5-7 received Compound 2 at 5, 11, and 25 mg/kg, respectively, qd x 21. Dosing solutions were prepared weekly by dissolving the appropriate amount of powder in 1% dimethylnitrosamine (DMA) in Labrasol® (vehicle) to yield a 5 mg/mL solution. The 5 mg/mL solution provided the 25 mg/kg dosage in a dosing volume of 5 mL/kg. An aliquot of the 5 mg/mL solution was diluted in the vehicle to concentrations of 2.2 and 1 mg/mL which provided the 11 and 5 mg/kg dosages, respectively, in a dosing volume of 5 mL/kg.

Tumors were measured using calipers twice per week. The study endpoint was defined as a mean tumor volume of 2000 mm³ in the control group or 22 days, whichever came first and the study was terminated on Day 22. Animals were weighed daily on Days 1-5, then twice per week until the completion of the study. The mice were observed frequently for overt signs of any adverse, treatment related (TR) side effects, and clinical signs were recorded when observed. Individual body weight loss was monitored as per protocol and any animal whose weight exceeded the limits for acceptable body weight loss was euthanized. Group mean body weight loss also was monitored as per protocol. Dosing was to be suspended in any group whose weight exceeded the limits for acceptable mean body weight loss. If mean body weight recovered, then dosing may be resumed in that group, but at a lower dosage or less frequent dosing schedule. Acceptable toxicity was defined as a group mean body weight loss of less than 20% during the study and not more than 10% treatment-related (TR) deaths. Any dosing regimen resulting in greater toxicity was considered above the maximum tolerated dose (MTD). A death was classified as TR if attributable to treatment side effects as evidenced by clinical signs and/or necropsy, or may also be classified as TR if due to unknown causes during the dosing period or within 14 days of the last dose. A death was classified as non-treatment-related (NTR) if there was no evidence that death was related to treatment side effects.

Treatment efficacy was determined using data from the final day. The MTV (n) (the median tumor volume for the number of animals, n) on the final day was determined for each group. Group 1 mice that received vehicle p.o. qd x 21 served as the control group for analysis of tumor growth inhibition (TGI) determined according to the formula %TGI = [1-(MTVdrug-treated/MTVcontrol)] x 100. Treatment efficacy was also determined from the incidence and magnitude of regression responses observed during the study. Treatment may cause partial regression (PR) or complete regression (CR) of the tumor in an animal. In a PR response, the tumor volume was 50% or less of its Day 1 volume for three consecutive measurements during the course of the study, and equal to or greater than 13.5 mm³ for one or more of these three measurements. In a CR response, the tumor volume was less than 13.5 mm³ for three consecutive measurements during the course of the study.

On Day 22, the MTV for Group 1 was 1421 mm³, with a range of 650 to 2890 mm³ (Figures 11A and 11B).

Response to Treatment with Compound 1 (Groups 2-4): On Day 22, the MTVs for Groups 2-4 were 446, 288, and 63 mm³, respectively, which corresponded to TGIs of 69, 80, and 96% (Figures 11A and 11B). The dose-related TGIs attained the threshold for potential therapeutic activity but only the 11 and 25 mg/kg regimens were significantly different from controls (Group 1 vs. 2, *P* > 0.05; Group 1 vs. 3, *P* < 0.05; Group 1 vs. 4, *P* < 0.001). There were no regression responses recorded in Groups 2 and 3, whereas Group 4 produced seven PRs. Median tumor growth for all three groups was delayed compared with that for controls (Figure 11A).

Response to Treatment with Compound 2 (Groups 5-7): Groups 5-7 received Compound 2 at 5, 11, and 25 mg/kg, respectively, p.o. qd x 21. On Day 22, the MTVs for Groups 5-7 were 320, 108, and 40 mm³, respectively, which corresponded to TGIs of 77, 92, and 97% (Figure 10(A-B)). The dose-related TGIs attained the threshold for potential therapeutic activity but only the 11 and 25 mg/kg regimens were significantly different from controls (Group 1 vs. 5, *P* > 0.05; Group 1 vs. 6, *P* < 0.01; Group 1 vs. 7, *P* < 0.001). There were no regression responses recorded in Group 5, however Group 6 produced three PRs and Group 7 produced eight PRs. Median tumor growth for all three groups was delayed compared with that for controls (Figure 10(A)).

Side Effects: Figure 11C shows percent mean body weight (BW) changes from Day 1 for each group. No TR deaths were assessed in the study and maximum mean BW losses were within acceptable limits for all groups.

This MV4-11 xenograft study demonstrates utility of the compounds of Formula I as potential anti-cancer therapy. The strong tumor regression responses are particularly impressive in light of the observed moderate body weight loss and no treatment-related deaths.

### Example 9 - Crystal Structures

PDK1 (residues 51-360) was expressed in *E. coli* as a GST fusion protein, purified by GSH affinity chromatography, cleaved, dialyzed, concentrated. PDK1 51-360 was mixed with compound and cocrystals were generated using the hanging drop vapor diffusion methodology.

Figures 12A through 12E illustrate the different PDK1-ligand structures observed for a compound of the invention as contrasted against ATP and the GSK2334470 and BX-320 PDK1 inhibitors. 12A): Crystal structure of Compound 3, a representative compound of Formula I, bound to PDK1. Compound 3 binds in the purine pocket of ATP and reaches deep into the core of the protein, occupying the binding pocket for the Phe of the DFG loop that is reoriented to the surface of the protein with Phe lying up against the compound. 12B): Overlay of PDK1 bound to ATP (PDB code 1h1w) and Compound 3. To a large extent, the two protein back-bones overlay closely, but significant perturbations are seen in the N-terminal (upper) lobe of the protein. Key conformations that are unique to ATP and Compound 3 are shown in lighter and darker grey, respectively. The two different locations of Phe/DFG loop are indicated. In the Compound 3 bound structure, the Phe residue resides in the area occupied by the phosphates of ATP in the ATP bound structure. In contrast, the Phe residue points toward the core of the protein in the ATP bound structure, an area that is occupied by the Compound 3 compound. Thus, ATP and Compound 3 bind two mutually exclusive conformations of PDK1. It is also seen that binding of Compound 3 causes a deformation of the αB and αC helices of the PDK1 protein relative to the ATP bound structure. 12C): Overlay of PDK1 structures with Compound 3 and GSK2334470 (GSK, lighter grey). The differences between the Compound 3 and GSK2334470-bound structures are primarily localized to the N-terminal (upper) lobe. Compound 3 occupies an area that is unique to the compounds of the invention and not accessed by GSK2334470. The location of the αB and αC helices is different when compared to GSK2334470 bound structure, which closely resembles the ATP bound structure 12D): Overlay of PDK1 structures with Compound 3 and BX-320 (darker grey). Compound 3 occupies an area of PDK1 that is unique to the compounds of the invention, causing a conformational change in PDK1 that displaces the DFG-loop and the αB and αC helices. Although the space occupied by the BX-320 compound is itself visibly different from GSK2334470, the PDK1-bound structures with these two compounds are substantially identical and closely resembles the ATP-bound structure. 12E) Top view of N-terminal kinase domain with Compound 3, GSK2334470, and BX-320. All three compounds occupy the pocket that binds purine of ATP. Otherwise, the compounds occupy different regions with BX-320 (Figure 12E) and GSK2334470 lying along the surface of the protein with only the compounds of Formula I reaching in to the core of the protein, where the compounds disrupt the interactions that hold and the αB and αC helices in their native conformation, as defined by the ATP-bound structure. This is important, as the αB and αC helices play a role in substrate binding and in stabilizing the catalytically active conformation of the kinase.

### Example 10 - Fluorescence PIF-tide binding assay

Peptide-binding assays were conducted with the following probes: FITC-REPRILSEEEQEMFRDFDYIADWC (SEQ ID NO.: 2), or FITC-EEQEMFRDFDYIADW (SEQ ID NO.: 3) (custom synthesis). Full-length phosphorylated PDK1 was incubated with the labeled peptides for 1 hr in 10 mM Tris (pH 7.5) containing 10 mM MgCl₂, 0.01% Triton X-100, and 1 mM DTT in the presence of compound or DMSO (0.1% final conc). Fluorescence polarization was measured using a Tecan Infinite F500 plate reader with λₑₓ = 535 nm, λₑₘ₁ = 590 nm.

FRET-based peptide-binding assays were conducted with FITC-REPRILSEEEQEMFRDFDYIADWC (SEQ ID NO.: 2). Full-length phosphorylated PDK1 was incubated with the labeled peptides and 0.4 nM anti-6His-Tb cryptate antibody (Cisbio Bioassays) for 1 hr in 10 mM Tris (pH 7.5) containing 10 mM MgCl₂, 0.01% Triton X-100, 0.01% casein and 1 mM DTT in the presence of compound or DMSO (0.1% final conc).

Figure 13A shows an overlay of PDK1 bound to Compound 3 (medium grey) and the comparator compounds GSK2334470 (lighest grey) and BX-320 (darkest grey) with a view of the PIF-tide binding pocket (black circle) at the top of the N-terminal lobe of PDK1. As can been seen, the representative compound of Formula I (medium grey) pushes out the αB and αC helices and thereby perturbs the structure of the PIF-binding pocket. To test this experimentally, a binding assay to measure PIF-tide binding to PDK1 was developed. In this assay, a compound that binds to the ATP-binding pocket of PDK1 without perturbing the PIF-pocket will show no effect of PIF-tide binding as measured by fluorescence polarization, as illustrated in Figure 13B (left half). However, a compound that binds to the ATP-binding pocket of PDK1 and perturbs the PIF-pocket will show reduced PIF-tide binding as measured by fluorescence polarization, as illustrated in Figure 13B (right half). The results from these binding studies, using PDK1 saturating amounts of compound, are shown in Figure 13C: As expected, Figure 13C) shows that BX-795 (a PDK1 inhibitor which closely resembles and has the same binding mode as BX-320) shows no interference with PIF-tide binding, while all three compounds of Formula I (Compound 3, Compound 1, and Compound 2) tested show significant inhibition of PIF-tide binding (the residual background signal is attributed to non-specific binding of PIF-tide). Thus, the compounds of Formula I can interfere with PIF-dependent substrate interactions in addition to inhibition of kinase activity. In Figure 13D data for a second method of monitoring the effect of compounds on PIF-tide binding to PDK1 are shown. In this assay, FRET between a Tb chelate bound to an antibody recognizing the 6His tag on PDK1 and the FITC-labeled PIF-tide is monitored. The pan-kinase inhibitor staurosporine, which does not disrupt the PIF-tide pocket, does not inhibit FRET between PDK1 and PIF-tide, while Compound 1 and Compound 2 show near complete inhibition at 10 nM compound. In Figure 6 (C), Compound 2 showed 90% P-RSK2 and 40% P-PDK1 inhibition at 4 hrs, while GSK2334470 only showed a modest 20% P-RSK2 inhibition despite showing 50% P-PDK1 inhibition (at 24 hrs), indicating that RSK2 phosphorylation is more sensitive to inhibition of PDK1 by Compound 2 than by GSK-2334470. The perturbation of the PIF-pocket and thus PIF-mediated RSK2 substrate binding could explain this enhanced sensitivity to Compound 2. This is a unique and novel aspect of the compounds of the invention that has not been observed for PDK1 catalytic inhibitors and may be of crucial importance for the enhanced cell-based potency of the compounds of the invention and their utility as anti-cancer drugs.

## Claims

1. A compound selected from the group consisting of: 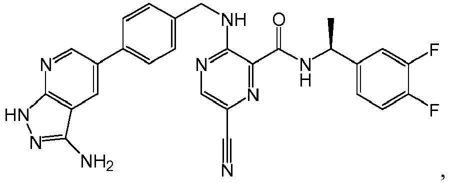 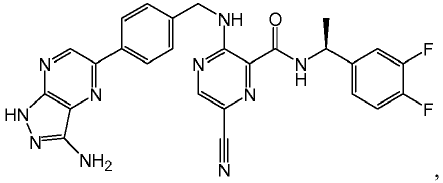 and 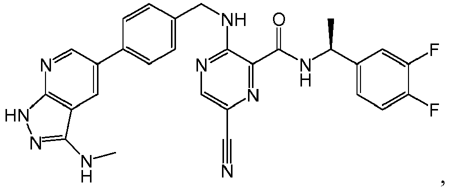 and pharmaceutically acceptable salts thereof, for use in a method of treating cancer in a subject in need thereof, in which cancer growth or survival is dependent on a PDK1-PIF-mediated substrate interaction, the method comprising administering to said subject a therapeutically effective amount of the compound or pharmaceutically acceptable salt thereof.

2. The compound for use of claim 1, in which the cancer is a hematologic cancer selected from the group consisting of leukemias, lymphomas, and myelomas.

3. The compound for use of claim 2, in which the hematologic cancer is selected from anaplastic large-cell lymphoma, non-Hodgkin's lymphoma, Hodgkin's lymphoma, B-cell lymphoma, T-cell lymphoma, mantle cell lymphoma, histiocytic lymphoma, T-cell leukemia, chronic lymphocytic leukemia, multiple myeloma, chronic myelogenous leukemia, acute lymphocytic (lymphoblastic) leukemia, acute myelogenous leukemia, acute myeloblastic leukemia, and plasma cell leukemia.

4. A pharmaceutical composition for use in treating cancer in a subject, in which the growth or proliferation of the cancer is dependent on a PDK1-PIF-mediated substrate interaction, comprising a formulation including a compound selected from the group consisting of: 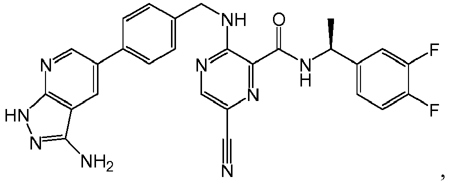 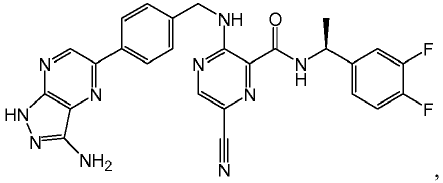 and 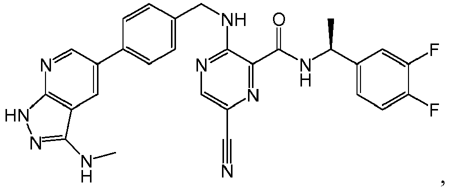 and pharmaceutically acceptable salts thereof; and
a pharmaceutically acceptable carrier.

5. A pharmaceutical composition for use in a combinational therapy of treating cancer in a subject, in which the growth or proliferation of the cancer is dependent on a PDK1-PIF-mediated substrate interaction, comprising a formulation including a compound selected from the group consisting of: 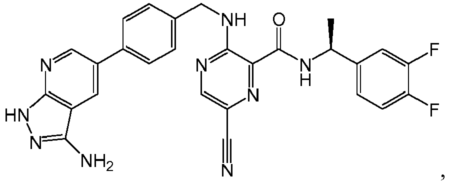 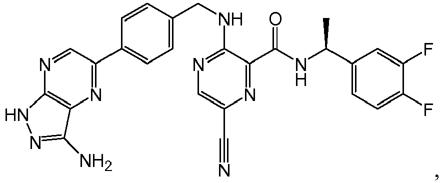 and 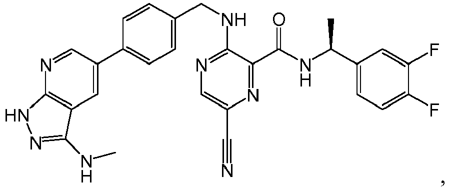 and pharmaceutically acceptable salts thereof; and
a pharmaceutically acceptable carrier, wherein the combinational therapy further comprises an effective amount of a second anti-cancer agent.

## Patentansprüche

1. Verbindung, die ausgewählt ist aus der Gruppe bestehend aus: 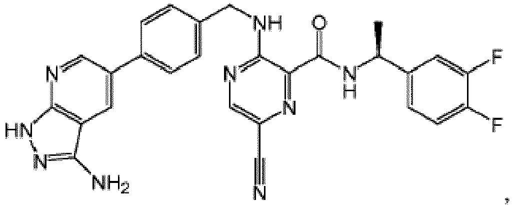 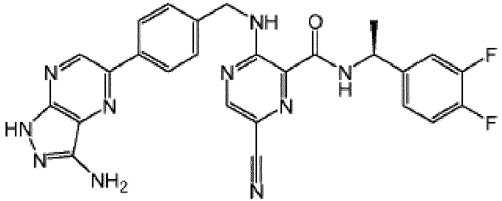 und 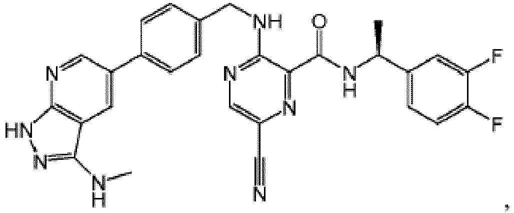 und pharmazeutisch annehmbare Salze davon zur Verwendung in einem Verfahren zur Behandlung einer Krebserkrankung bei einem Probanden, der dieser bedarf, wobei Krebswachstum oder -überleben von einer PDK1-PIF-vermittelten Substratinteraktion abhängt, wobei das Verfahren ein Verabreichen einer therapeutisch wirksamen Menge der Verbindung oder des pharmazeutisch annehmbaren Salzes davon an den Probanden umfasst.

2. Verbindung zur Verwendung nach Anspruch 1, wobei die Krebserkrankung eine hämatologische Krebserkrankung ist, die aus der Gruppe bestehend aus Leukämien, Lymphomen und Myelomen ausgewählt ist.

3. Verbindung zur Verwendung nach Anspruch 2, wobei die hämatologische Krebserkrankung aus anaplastischem großzelligem Lymphom, Non-Hodgkin-Lymphom, Hodgkin-Lymphom, B-Zell-Lymphom, T-Zell-Lymphom, Mantelzell-Lymphom, histiozytischem Lymphom, T-Zell-Leukämie, chronischer lymphatischer Leukämie, multiplem Myelom, chronischer myeloischer Leukämie, akuter lymphatischer (lymphoblastischer) Leukämie, akuter myeloischer Leukämie, akuter myeloblastischer Leukämie und Plasmazell-Leukämie ausgewählt ist.

4. Pharmazeutische Zusammensetzung zur Verwendung bei der Behandlung einer Krebserkrankung bei einem Probanden, wobei das Wachstum oder die Proliferation der Krebserkrankung von einer PDK1-PIF-vermittelten Substratinteraktion abhängt, umfassend eine Formulierung, die eine Verbindung, die ausgewählt ist aus der Gruppe bestehend aus: 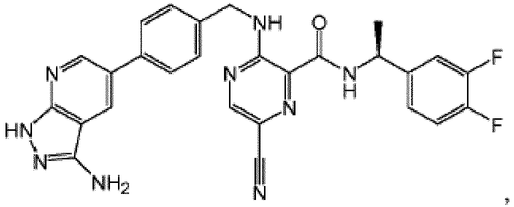 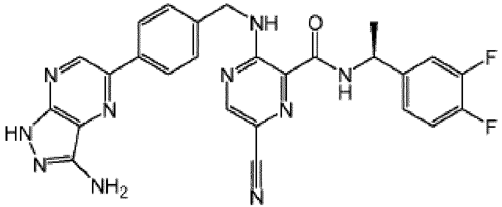 und 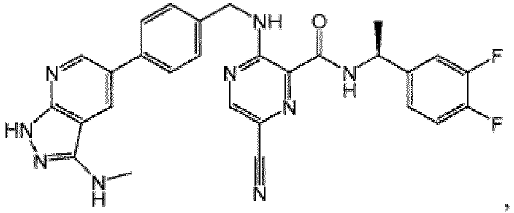 und pharmazeutisch annehmbare Salze davon und
einen pharmazeutisch annehmbaren Trägerstoff enthält.

5. Pharmazeutische Zusammensetzung zur Verwendung in einer Kombinationstherapie zur Behandlung einer Krebserkrankung bei einem Probanden, wobei das Wachstum oder die Proliferation der Krebserkrankung von einer PDK1-PIF-vermittelten Substratinteraktion abhängt, umfassend eine Formulierung, die eine Verbindung, die ausgewählt ist aus der Gruppe bestehend aus: 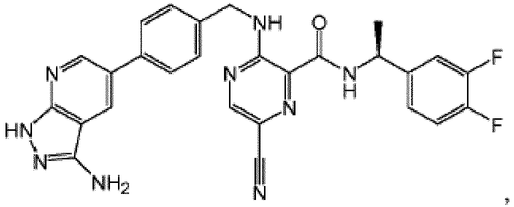 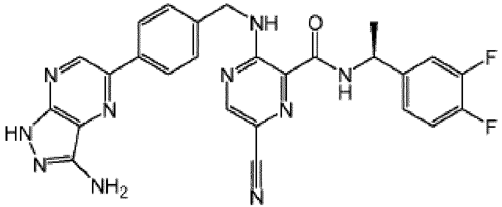 und 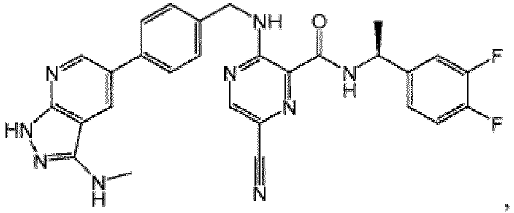 und pharmazeutisch annehmbare Salze davon und
einen pharmazeutisch annehmbaren Trägerstoff enthält, wobei die Kombinationstherapie weiterhin eine wirksame Menge eines zweiten Antikrebsmittels umfasst.

## Revendications

1. Composé choisi dans le groupe constitué par : 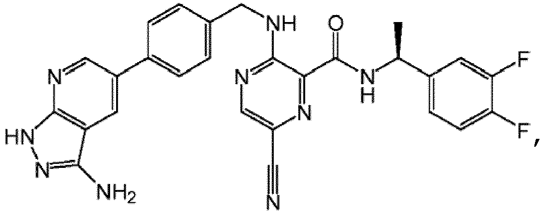 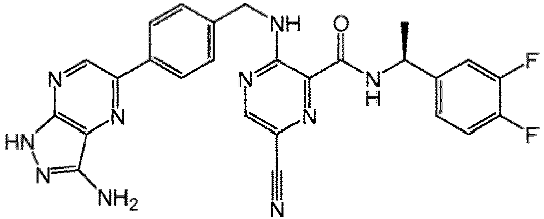 et 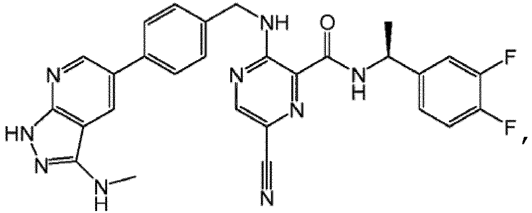 et les sels pharmaceutiquement acceptables de ceux-ci, destiné à être utilisé dans une méthode de traitement de cancer chez un sujet qui en a besoin, la croissance ou la survie du cancer étant dépendante d'une interaction avec des substrats médiée par PDK1-PIF, la méthode comprenant l'administration audit sujet d'une quantité thérapeutiquement efficace du composé ou du sel pharmaceutiquement acceptable de celui-ci.

2. Composé destiné à être utilisé selon la revendication 1, le cancer étant un cancer hématologique choisi dans le groupe constitué par les leucémies, les lymphomes et les myélomes.

3. Composé destiné à être utilisé selon la revendication 2, le cancer hématologique étant choisi entre un lymphome anaplasique à grandes cellules, un lymphome non hodgkinien, un lymphome hodgkinien, un lymphome B, un lymphome T, un lymphome à cellules du manteau, un lymphome histiocytaire, une leucémie à lymphocytes T, une leucémie lymphocytaire chronique, un myélome multiple, une leucémie myéloïde chronique, une leucémie lymphocytaire (lymphoblastique) aiguë, une leucémie myéloïde aiguë, une leucémie myéloblastique aiguë et une leucémie à plasmocytes.

4. Composition pharmaceutique destinée à être utilisée en traitement de cancer chez un sujet, la croissance ou la prolifération du cancer étant dépendante d'une interaction avec des substrats médiée par PDK1-PIF, comprenant une formulation renfermant un composé choisi dans le groupe constitué par : 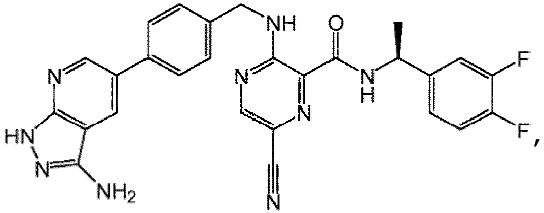 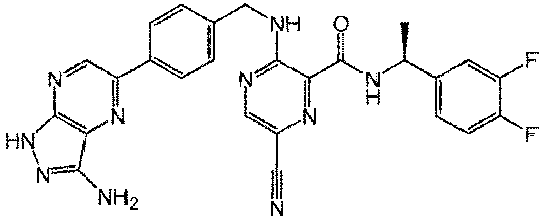 et 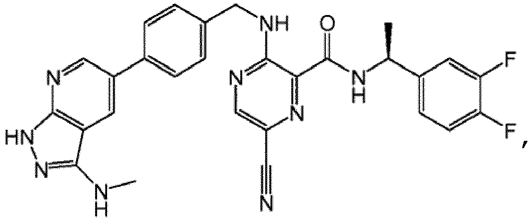 et les sels pharmaceutiquement acceptables de ceux-ci ; et
un véhicule pharmaceutiquement acceptable.

5. Composition pharmaceutique destinée à être utilisée dans une polythérapie de traitement de cancer chez un sujet, la croissance ou la prolifération du cancer étant dépendante d'une interaction avec des substrats médiée par PDK1-PIF, comprenant une formulation renfermant un composé choisi dans le groupe constitué par : 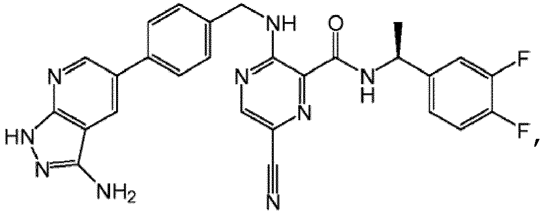 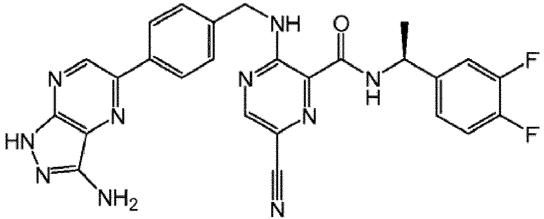 et 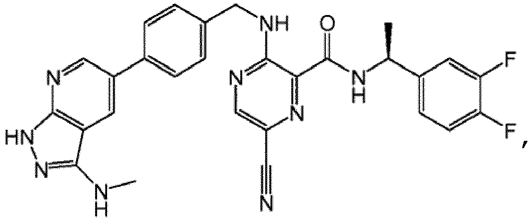 et les sels pharmaceutiquement acceptables de ceux-ci ; et
un véhicule pharmaceutiquement acceptable,
la polythérapie comprenant en outre une quantité efficace d'un second agent anticancéreux.
